(19) **Europäisches Patentamt**
**European Patent Office**
**Office européen des brevets**

(11) **EP 4 147 047 B1**

(12) **EUROPEAN PATENT SPECIFICATION**

(45) Date of publication and mention
of the grant of the patent:
**27.08.2025 Bulletin 2025/35**

(21) Application number: **21724267.6**

(22) Date of filing: **06.05.2021**

(51) International Patent Classification (IPC):
**G01N 33/543** (2006.01)   **G01N 33/569** (2006.01)
**G01N 33/574** (2006.01)   **G01N 33/68** (2006.01)
**G16B 30/10** (2019.01)

(52) Cooperative Patent Classification (CPC):
**G01N 33/56977; G01N 33/54366; G01N 33/574;**
**G01N 33/6878; G16B 30/10; G16B 35/20;**
G01N 2333/70539; G16H 20/10; G16H 50/20;
Y02A 90/10

(86) International application number:
**PCT/EP2021/061981**

(87) International publication number:
**WO 2021/224383 (11.11.2021 Gazette 2021/45)**

(54) **IDENTIFICATION OF TUMOR-SPECIFIC ANTIGENS**

IDENTIFIZIERUNG TUMORSPEZIFISCHER ANTIGENE

IDENTIFICATION D'ANTIGÈNES SPÉCIFIQUES DE TUMEURS.

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **07.05.2020 GB 202006760**

(43) Date of publication of application:
**15.03.2023 Bulletin 2023/11**

(73) Proprietor: **University of Helsinki**
**00014 University of Helsinki (FI)**

(72) Inventors:
• **CERULLO, Vincenzo**
**00014 University of Helsinki Helsinki (FI)**
• **CAPASSO, Cristian**
**00014 University of Helsinki Helsinki (FI)**
• **SIKANEN, Tiina**
**00014 University of Helsinki Helsinki (FI)**
• **FEOLA, Sara**
**00014 University of Helsinki Helsinki (FI)**
• **TÄHKÄ, Sari**
**00014 University of Helsinki Helsinki (FI)**
• **CHIARO, Jacopo**
**00014 University of Helsinki Helsinki (FI)**

(74) Representative: **Symbiosis IP Limited**
**Unit 5C**
**Sbarc I Spark Building**
**Maindy Road**
**Cardiff CF24 4HQ (GB)**

(56) References cited:
**WO-A1-2020/236850    WO-A1-2020/239609**
**US-A1- 2005 239 146    US-A1- 2017 016 075**

• **CAPASSO C ET AL: "Homology between cancer**
**and viral epitopes as criteria to design improved**
**cancer vaccines", ANNALS OF ONCOLOGY, vol.**
**28, no. S11, 1 December 2017 (2017-12-01),**
**XP055814582, Retrieved from the Internet**
**<URL:https://www.annalsofoncology.org/article/**
**S0923-7534(19)32823-6/pdf>**
• **PAMELA C. ROSATO ET AL: "Virus-specific**
**memory T cells populate tumors and can be**
**repurposed for tumor immunotherapy", NATURE**
**COMMUNICATIONS, vol. 10, no. 1, 1 February**
**2019 (2019-02-01), XP055569651, DOI: 10.1038/**
**s41467-019-08534-1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- E. F. FRITSCH ET AL: "HLA-Binding Properties of Tumor Neoepitopes in Humans", CANCER IMMUNOLOGY RESEARCH, vol. 2, no. 6, 3 March 2014 (2014-03-03), US, pages 522 - 529, XP055420002, ISSN: 2326-6066, DOI: 10.1158/2326-6066.CIR-13-0227
- CHONG CHLOE ET AL: "High-throughput and Sensitive Immunopeptidomics Platform Reveals Profound Interferon[gamma]-Mediated Remodeling of the Human Leukocyte Antigen (HLA) Ligandome", MOLECULAR & CELLULAR PROTEOMICS, vol. 17, no. 3, 5 October 2017 (2017-10-05), US, pages 533 - 548, XP055814703, ISSN: 1535-9476, Retrieved from the Internet <URL:https://www.ncbi.nlm.nih.gov/pmc/articles/PMC5836376/pdf/zjw533.pdf> DOI: 10.1074/mcp.TIR117.000383
- "Current Protocols in Immunology", 1 February 2013, JOHN WILEY & SONS, INC., Hoboken, NJ, USA, ISBN: 978-0-471-14273-7, article JOHN SIDNEY ET AL: "Measurement of MHC/Peptide Interactions by Gel Filtration or Monoclonal Antibody Capture", XP055365398, DOI: 10.1002/0471142735.im1803s100

- RODRÍGUEZ-RUIZ I. ET AL: "Protein separation under a microfluidic regime", ANALYST, vol. 143, no. 3, 1 January 2018 (2018-01-01), UK, pages 606 - 619, XP055814684, ISSN: 0003-2654, Retrieved from the Internet <URL:https://pubs.rsc.org/en/content/articlepdf/2018/an/c7an01568b> DOI: 10.1039/C7AN01568B
- FEOLA SARA ET AL: "PeptiCHIP: a novel microuidic-based chip platform for tumour antigen landscape identication", RESEARCH SQUARE, 3 March 2021 (2021-03-03), XP055814535, Retrieved from the Internet <URL:https://www.researchsquare.com/article/rs-156531/v1.pdf> [retrieved on 20210616], DOI: 10.21203/rs.3.rs-156531/v1

Remarks:
  The complete document including Reference Table(s) and the Sequence Listing(s) can be downloaded from the EPO website

## Description

### Field of the Invention

The invention concerns a device for tumour-specific antigen identification, a method for tumour-specific antigen identification; and a method of stratifying patients for checkpoint inhibitor cancer treatment using said device and/or said method.

### Background of the Invention

CD8$^+$ T-cells have a key role in the detection and elimination of cells that present abnormal peptides on their surface as a result of pathogenic infection, such as viral infection, or malignant transformation. The cross-reactivity of the T-cell receptor (TCR) means T-cells can recognize an incredibly large variety of different target peptides. This phenomenon allows a relatively small number of T-cells to recognize multiple pMHC (peptide:Major Histocompatibility Complex) molecules that are representative of abnormal cells and so potentially health or life threatening.

However, an undesirable side effect of this mechanism is that an immune response directed against a pathogen might overcome the tolerance threshold for a highly homologous self-antigen, causing a deleterious off-target effect that is mediated by the cross-reactivity of T-cells; a process known as molecular mimicry. In the field of autoimmunity, the potentially deleterious consequences of such homology between self and pathogenic peptides is well-known, however, it has not been explored in cancer.

Indeed, it has been thought that the best prognostic markers for successful outcome in cancer immunotherapeutic treatment is a high tumor antigen mutational rate and abundant T-cell infiltration. This thinking is based upon the fact that a tumor that has a high number of mutations will have a higher chance of being recognized and eliminated by infiltrating T-cells.

Nevertheless, some studies have shown that the qualitative properties of tumor antigens might be more important than their quantity. Furthermore, it has been observed that anti-viral T-cells populate the tumor microenvironment [28], however whether their role is active or just bystander is still unclear. Additionally, Capasso et al. Annals of Oncology, Vol 28, S11, 1 Dec 2017, teach a cancer vaccine based on the similarity of a tumour peptide with a viral peptide, using an in silico platform, to engage a wide range of of lymphocyties. and HLA-affinity and sequence homology are evaluated.

Onco-immunology and immunotherapy have completely changed the way cancer is being treated over the last decade, particularly when using checkpoint inhibitors (ICIs) where outstanding clinical results have been seen but, unfortunately, only in a small number of patients. It is becoming clear that immunotherapy with ICIs will only fully work when targeting specific tumor antigens. However, at the moment there is not an easy and rapid method to identify these tumor antigens.

Herein, we hypothesized that tumors might present peptides that share a high degree of homology scoring with pathogen-derived peptides, in particular, viral peptides and this might enable pathogenically-generated, cross-reactive T-cells to recognize and kill tumor cells.

To this end, we have developed a bioinformatic device called HEX (Homology Evaluation of Xenopeptides) to automatically and very simply identify tumor antigens that are highly similar to viral peptides. Using this device, we have observed that anti-viral T-cell immunity, mediated by a peptide with high homology scoring with a cancer antigen, can also actively control tumor growth in both a prophylactic and a therapeutic setting. This observation demonstrates the cross-reactivity of activated T-cells against both homologous viral-derived peptides and tumor-derived peptides.

Subsequently, we have also found that a humoral response to cytomegalovirus (CMV) was able to stratify the response of melanoma patients' to checkpoint inhibitor therapy (anti-PD1). Indeed, peptides homologous with CMV and melanoma, identified through use of the device HEX, were found to foster Inf-g release in Pheripheral Blood Mononuclear Cells (PBMCs) from CMV seropositive melanoma patients.

### Statements of the Invention

The invention protected herein is as described in the accompanying claims and reproduced below.

According to a first aspect of the invention there is provided a device for tumour-specific antigen identification comprising:

a microfluidic device comprising at least one flow-through channel containing a plurality of micropillars arranged in an array to which there is attached at least one molecule, or at least one complex, that has bound thereto at least one anti-Major Histocompatibility Complex antibody or at least one anti-pan-Human Leukocyte Antigen (HLA) antibody whereby a peptide:Major Histocompatibility Complex (pMHC) in a sample flowing through said channel can be extracted from said sample using said at least one antibody; and

a processor adapted for identifying said peptide bound to said Major Histocompatibility Complex and comparing said peptide of said bound pMHC with a library of pathogen-derived peptide antigens to determine if said peptide bound or that was bound to said Major Histocompatibility Complex shows homology, by way of a comparison of sequence structures, with at least one pathogenic antigen, or part thereof, and where greater than 60% homology exists; indicating said peptide bound or that was bound to said Major Histocompatibility Complex is a tumour-specific antigen for use in cancer therapy.

Reference herein to a anti-pan-HLA antibody is to an antibody that recognises or has specificity for any of the different HLAs present in mammals, in particular humans.

More ideally still, said antibody recognises or has specificity for a particular HLA type such as MHC-I and selected from at least one of the following group: MHC class I A, B, and C.

Additionally or alternatively, said antibody recognises or has specificity for a particular HLA type such as MHC-II selected from at least one of the following group: MHC class II DP, DM, DO, DQ, and DR.

Yet more preferably still said antibody is anti-human.

In a preferred embodiment of the invention said molecule or complex is a complex of thiol and alkyl ("ene") functional groups, ideally, involving the stoichiometric ratio of 1.5 to 1.0 within the range of 0.15:0.1-1500:1000 (tetrathiol: triallyl).

More ideally still, the fabrication of the device is based on UV-initiated photoreaction between the thiol and alkyl ("ene") functional groups. In yet a further preferred embodiment of the invention the device is made by UV photopolymerization with biotin-PEG4-alkyne (Sigma, 764213). This is followed by reaction with avidin: streptavidin. Although, it is within the scope of the invention to attach the avidin to the biotic before attaching the biotic to the pillar and vice versa.

In still yet a further preferred embodiment said device is functionalised by reacting said antibody with said streptavidin. Ideally, more than one antibody is reacted with said streptavidin whereby each complex functionalised with streptavidin carries a plurality, such 2 or 3, of said antibodies such as a plurality of anti-pan-HLA antibodies.

Typically, although not claimed, the micropillar array was preconditioned with protein, such as bovine serum albumin (BSA) (ideally 100 $\mu$g/mL in 15mM PBS, 10 min incubation) after streptavidin functionalization and before antibody binding.

Typically, although not claimed, said micropillars are made of a same or similar composition to that which is used in conventional microfluidic devices e.g. for implementing immobilized enzyme reactions.

According to a further aspect of the invention there is provided a method for tumour-specific antigen identification comprising:

i) dissolving or suspending a sample of tumour in a fluid;

ii) passing said fluid through a microfluidic device for tumour-specific antigen identification comprising: at least one flow-through channel containing a plurality of micropillars arranged in an array to which there is attached at least one molecule, or at least one complex, that has bound thereto at least one anti-Major Histocompatibility Complex antibody or at least one anti-pan-Human Leukocyte Antigen antibody whereby a peptide:Major Histocompatibility Complex (pMHC) in a sample flowing through said channel can be extracted from said sample using said at least one antibody.

iii) binding to said at least one antibody at least one pMHC in said sample;

iv) removing said at least one bound pMHC of part iii) from said device;

v) comparing said peptide of said bound pMHC with a library of pathogen-derived antigens to determine if said peptide shows homology, by way of a comparison of sequence structures, with at least one pathogenic antigen, or part thereof, and where greater than 60% homology exists;

vi) identifying said peptide as a tumour-specific antigen for use in cancer therapy.

In a preferred embodiment of any aspect of the invention said homology may be any one of the following percentages: 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99 and 100%.

Reference herein to homology includes reference to a comparison of sequence structures in part v) above using one of: homology as defined herein; or identity which is determined by the number of identical residues over a defined length in a given alignment; or similarity which is determined by the number of identical residues or conservative substitutions having similar physicochemical properties over a defined length in a given alignment.

In a preferred method of the invention said library of human pathogen-derived antigens comprises a curated library of known pathogenic antigens, ideally derived from pathogenic viruses, or more suitably the proteomes thereof, which viruses infect mammals, ideally humans, such as any one or more, or ideed any combination, of the following viruses:

Abyssoviridae; Ackermannviridae; Actantavirinae; Adenoviridae; Agantavirinae; Aglimvirinae; Alloherpesviridae; Alphaflexiviridae; Alphaherpesvirinae; Alphairidovirinae; Alphasatellitidae; Alphatetraviridae; Alvernaviridae; Amalgaviridae; Amnoonviridae; Ampullaviridae; Anelloviridae; Arenaviridae; Arquatrovirinae; Arteriviridae; Artoviridae; Ascoviridae; Asfarviridae; Aspiviridae; Astroviridae; Autographivirinae; Avsunviroidae; Avulavirinae; Bacilladnaviridae; Baculoviridae; Barnaviridae; Bastillevirinae; Bclasvirinae; Belpaoviridae; Benyviridae; Betaflexiviridae; Betaherpesvirinae; Betairidovirinae; Bicaudaviridae; Bidnaviridae; Birnaviridae; Bornaviridae; Botourmiaviridae; Brockvirinae; Bromoviridae; Bullavirinae; Caliciviridae; Calvusvirinae; Carmotetraviridae; Caulimoviridae; Ceronivirinae; Chebruvirinae; Chordopoxvirinae; Chrysoviridae; Chuviridae; Circoviridae; Clavaviridae; Closteroviridae; Comovirinae; Coronaviridae; Corticoviridae; Crocarterivirinae; Cruliviridae; Crustonivirinae; Cvivirinae; Cystoviridae; Dclasvirinae; Deltaflexiviridae; Densovirinae; Dicistroviridae; Endornaviridae; Entomopoxvirinae; Equarterivirinae; Eucampyvirinae; Euroniviridae; Filoviridae; Fimoviridae; Firstpapillomavirinae; Flaviviridae; Fuselloviridae; Gammaflexiviridae; Gammaherpesvirinae; Geminialphasatellitinae; Geminiviridae; Genomoviridae; Globuloviridae; Gokushovirinae; Guernseyvirinae; Guttaviridae; Hantaviridae; Hepadnaviridae; Hepeviridae; Herelleviridae; Heroarterivirinae; Herpesviridae; Hexponivirinae; Hypoviridae; Hytrosaviridae; Iflaviridae; Inoviridae; Iridoviridae; Jasinkavirinae; Kitaviridae; Lavidaviridae; Leishbuviridae; Letovirinae; Leviviridae; Lipothrixviridae; Lispiviridae; Luteoviridae; Malacoherpesviridae;; Mammantavirinae; Marnaviridae; Marseilleviridae; Matonaviridae; Mccleskeyvirinae Mclasvirinae; Medioniviridae; Medionivirinae; Megabirnaviridae; Mesoniviridae; Metaparamyxovirinae; Metaviridae; Microviridae; Mimiviridae; Mononiviridae; Mononivirinae; Mymonaviridae; Myoviridae; Mypoviridae; Nairoviridae; Nanoalphasatellitinae; Nanoviridae; Narnaviridae; Nclasvirinae; Nimaviridae; Nodaviridae; Nudiviridae; Nyamiviridae; Nymbaxtervirinae; Okanivirinae; Orthocoronavirinae; Orthomyxoviridae; Orthoparamyxovirinae; Orthoretrovirinae Ounavirinae; Ovaliviridae; Papillomaviridae; Paramyxoviridae; Partitiviridae; Parvoviridae; Parvovirinae; Pclasvirinae; Peduovirinae; Peribunyaviridae; Permutotetraviridae; Phasmaviridae; Phenuiviridae; Phycodnaviridae; Picobirnaviridae; Picornaviridae; Picovirinae; Piscanivirinae; Plasmaviridae; Pleolipoviridae; Pneumoviridae; Podoviridae; Polycipiviridae; Polydnaviridae; Polyomaviridae; Portogloboviridae; Pospiviroidae; Potyviridae; Poxviridae; Procedovirinae; Pseudoviridae; Qinviridae; Quadriviridae; Quinvirinae; Regressovirinae; Remotovirinae; Reoviridae; Repantavirinae; Retroviridae; Rhabdoviridae; Roniviridae; Rubulavirinae; Rudiviridae; Sarthroviridae; Secondpapillomavirinae; Secoviridae; Sedoreovirinae; Sepvirinae; Serpentovirinae; Simarterivirinae; Siphoviridae; Smacoviridae; Solemoviridae; Solinviviridae; Sphaerolipoviridae; Spinareovirinae; Spiraviridae; Spounavirinae; Spumaretrovirinae; Sunviridae; Tectiviridae; Tevenvirinae; Tiamatvirinae; Tobaniviridae; Togaviridae; Tolecusatellitidae; Tombusviridae; Torovirinae; Tospoviridae; Totiviridae;; Tristromaviridae; Trivirinae; Tunavirinae; Tunicanivirinae; Turriviridae; Twortvirinae; Tymoviridae; Variarterivirinae; Vequintavirinae; Virgaviridae; Wupedeviridae; Xinmoviridae; Yueviridae; and Zealarterivirinae.

More preferably the pathogenic antigen is a cytomegalovirus (CMV) antigen or an Epstein-Barr virus (EBV) antigen or, preferably, a Herpesvirus antigen, a Poxvirus antigen, a Hepadnavirus antigen, an Influenza virus antigen, a Coronavirus antigen, a Hepatitis virus antigen, a HIV antigen, or a Bunyavirus antigen.

Most preferably said virus is non-oncolytic i.e. its replication is not specifically restricted to cancer cells..

In an alternative embodiment, said virus is oncolytic i.e. capable of infecting and killing cancer cells by selective replication in tumour versus normal cells.

In a preferred method said comparing said peptide of said bound pMHC (peptide:Major Histocompatibility Complex) with a library of pathogenic antigens involves a plurality of scorings (peptide scoring and alignment scoring and similarity scoring and MHC-binding affinity scoring) to determine said homology or identity or similarity. Most preferably, said scoring is an homology scoring as described below.

In one embodiment, a matrix of rows (or columns) representing the amino acid position in the tumour peptide and columns (or rows) representing each of the 20 standard amino acids is generated and the amino acid positions of the tumour peptide are assigned the same high score and other positions are assigned the same low score as the pathogen-derived antigen/peptide. Tumour peptides with the highest degree of homology with a pathogen-derived antigen/peptide in terms of (in descending order of preference):

a) entire sequence structure (clearly a tumour peptide with 100% identity with a pathogen-derived peptide will recieve the highest score);
b) identity of key amino acids in hot-spot or key binding sites; and
c) the most number of of key amino acids in hot-spot or key binding sites

will receive the highest scores.

Alignments are computed pairwise between peptides, in the query (pathogen-derived) set against the tumour set, or *vice versa*. For a given pair of peptides, their alignment is calculated by summing the distance scores between pairs of amino acids in the same position. Scoring is weighted to prioritize similarity between more central amino acids in the peptide.

Additionally, MHC class I binding affinity predictions are made. Methods for achieving same are known in the art such as using NetMHC (NetMHC4.0 or NetMHCpan4.1.) via the Application programming interface of IEDB (http://tools.

iedb.org/main/tools-api/) and are then parsed and collated within the tool.

In an alternative embodiment of the invention, said comparing involves inputting lists of tumor peptides of 8 to 12 amino acides in length into the software. Firstly, the tool BLAST is employed to find Hits (similar sequences) in the library of pathogen-derived antigens. For this task the PAM30 tool is generally used, however both BLOSUM and PAM substitution matrices across several evolutionary distances are supported. Next, a pairwise refinement alignment using at least BLOSUM62 and, ideally also, a substitution matrix developed by Kim et al (described in BMC Bioinformatics. 2009;10:394. Published 2009 Nov 30. doi:10.1186/1471-2105-10-394 Derivation of an amino acid similarity matrix for peptide: MHC binding and its application as a Bayesian prior. Kim Y, Sidney J, Pinilla C, Sette A, Peters B.) is performed in order to refine the alignment in a position-weighted fashon. A pair of peptides with high similarity in the TCR-interacting area (the central section of the peptides) receive a higher similarity score. Finally, a MHC-binding affinity prediction can be produced for both the tumor and the viral cognate peptide using NetMHC4.0 or NetMHCpan4.1 as standalone command line tools. The results are ultimately automatically parsed and collated within the tool and a final score which summarize the above mentioned analysis is created.

The invention also concerns a checkpoint inhibitor (ICI).

The best characterized pathways for checkpoint inhibition are cytotoxic T-lymphocyte protein 4 (CTLA-4) pathway and programmed cell death protein 1 pathway (PD-1/PD-L1). Thus, the invention can be used in combination with at least one checkpoint modulator such as anti-CTLA-4, anti-PD1, or anti-PD-L1 molecules to counteract the immunosuppressive tumor environment and to cause a strong anti-immune response.

According to a yet further aspect of the invention there is provided a method of stratifying patients for checkpoint inhibitor cancer treatment comprising:

i) dissolving or suspending a sample of tumour from a patient in a fluid;
ii) passing said fluid through a microfluidic device for tumour-specific antigen identification comprising: at least one flow-through channel containing a plurality of micropillars arranged in an array to which there is attached at least one molecule, or at least one complex, that has bound thereto at least one anti-Major Histocompatibility Complex antibody or at least one anti-pan-Human Leukocyte Antigen antibody whereby a peptide:Major Histocompatibility Complex (pMHC) in a sample flowing through said channel can be extracted from said sample using said at least one antibody;
iii) binding to said at least one antibody at least one pMHC in said sample;
iv) removing said at least one bound pMHC of part iii) from said device;
v) comparing said peptide of said bound pMHC with a library of human pathogen- antigens to determine if said peptide shows homology, by way of a comparison of sequence structures, with at least one human pathogenic antigen, or part thereof, and where greater than 60% homology exists;
vi) identifying said peptide as a tumour-specific antigen; and
vii) when said pepide is found, stratifying said patient as suitable for treatment using an effective amount of at least a checkpoint inhibitor.

Reference herein to an "effective amount", is one that is sufficient to achieve a desired biological effect, such as, cancer cell death.

It is understood that the effective dosage will be dependent upon the age, sex, health, and weight of the recipient, kind of concurrent treatment, if any, frequency of treatment, and the nature of the effect desired. Typically, the effective amount is determined by those administering the treatment.

Most preferably the cancer referred to herein includes any one or more of the following cancers: nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, oligodendroglioma, neuroblastoma, meningioma, spinal cord tumor, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, esophagus cancer, gall bladder cancer, head cancer, eye cancer, neck cancer, kidney cancer, Wilms' tumor, liver cancer, Kaposi's sarcoma, prostate cancer, testicular cancer, Hodgkin's disease, non-Hodgkin's lymphoma, skin cancer, mesothelioma, multiple myeloma, ovarian cancer, endocrine pancreatic cancer, glucagonoma, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

In the claims which follow and in the preceding description of the invention, except where the context requires otherwise due to express language or necessary implication, the word "comprise", or variations such as "comprises" or "comprising" is used in an inclusive sense i.e. to specify the presence of the stated features but not to preclude the presence or addition of further features in various embodiments of the invention.

No admission is made that any reference constitutes prior art. Further, no admission is made that any of the prior art constitutes part of the common general knowledge in the art.

Preferred features of each aspect of the invention may be as described in connection with any of the other aspects.

Other features of the present invention will become apparent from the following examples. Generally speaking, the invention extends to any novel one, or any novel combination, of the features disclosed in this specification (including the accompanying claims and drawings). Thus, features, integers, characteristics, compounds or chemical moieties described in conjunction with a particular aspect, embodiment or example of the invention are to be understood to be applicable to any other aspect, embodiment or example described herein, unless incompatible therewith.

Moreover, unless stated otherwise, any feature disclosed herein may be replaced by an alternative feature serving the same or a similar purpose.

Throughout the description and claims of this specification, the singular encompasses the plural unless the context otherwise requires. In particular, where the indefinite article is used, the specification is to be understood as contemplating plurality as well as singularity, unless the context requires otherwise.

An embodiment of the present invention will now be described by way of example only with reference to the following wherein:

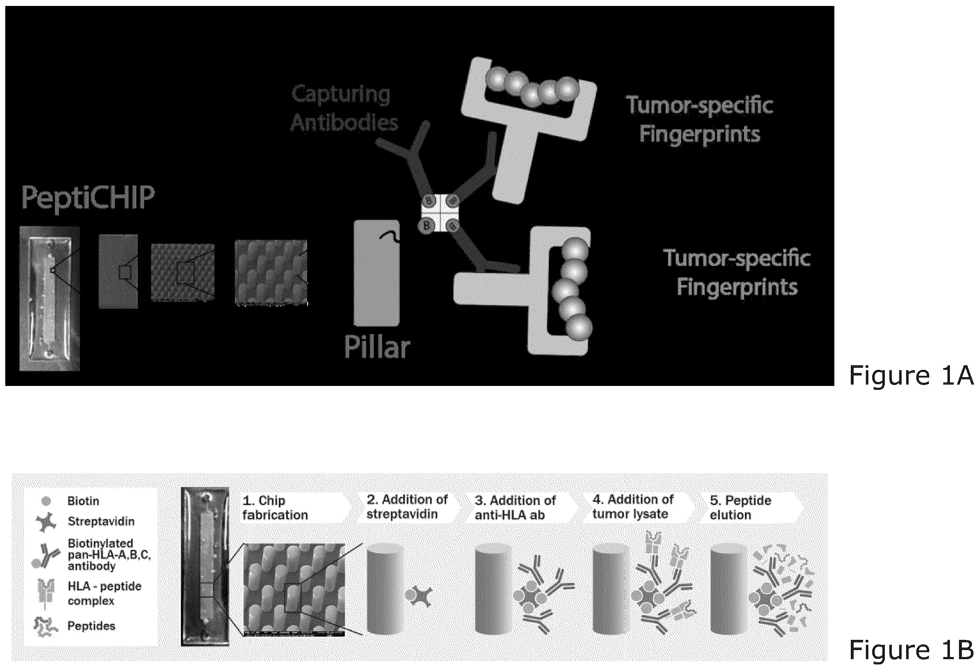

**Figure 1. [A] Inside PeptiCHIP.** PeptiCHIP inside consists of thousands of pillars that are coated with a linker to which is attached the Biotin. Biotin is then made to react with streptavidin that can then later react with three molecules of HLA-specific capturing antibody. **[B]. Microchip technology as a novel immunopurification platform for fast antigen discovery.**

A schematic overview describing the new microchip methodology developed. Thiol-ene microchips incorporating free surface thiols are derivatized with biotin-PEG4-alkyne thiolene (Step1) and functionalized with a layer of streptavidin (Step2) after which a biotinylated pan-HLA antibody is immobilized on the micropillar surface (Step3) and cell lysate is loaded into the microchip (Step4). After adequate incubation time and washing steps, the HLA molecules are eluted by adding 7% acetic acid (Step5).

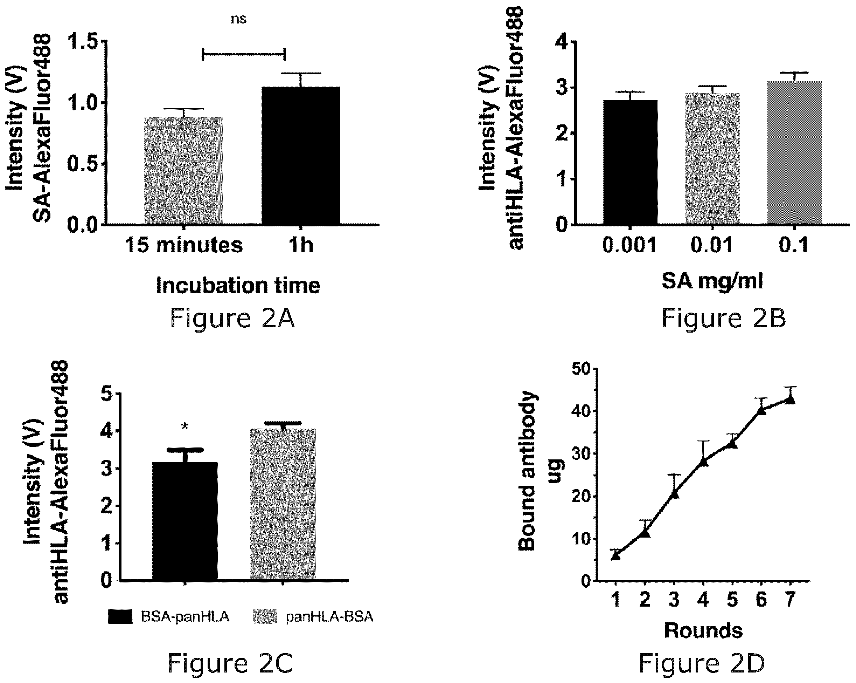

**Figure 2. Characterization of the selectivity of the microchip functionalization with biotinylated pan-HLA antibody.**

A) Binding efficacy of AlexaFluor 488-streptavidin on thiol-ene micropillars precoated with biotin-PEG4-alkyne at two different streptavidin incubation times (15 min and 1h). B) The effect of streptavidin (nonfluorescent) concentration on the amount of immobilized biotinylated pan-HLA antibody quantitated through AlexaFluor 488-labeled secondary antibody. C) The effect of BSA incubation on the amount of immobilized biotinylated pan-HLA antibody quantitated through AlexaFluor 488-labeled secondary antibody. The efficiency of BSA in blocking nonspecific binding sites was assessed by preconditioning the micropillar array with BSA either before (BSA-panHLA) or after (panHLA-BSA) immobilization of the biotinylated pan-HLA antibody. D) The total amount of biotinylated pan-HLA antibody bound onto a single chip as a function of loading cycles. For each cycle, a fresh batch of the same (constant) pan-HLA antibody concentration was used. Significance was assessed by two-tailed unpaired Student's t-test, * $p<0.05$.

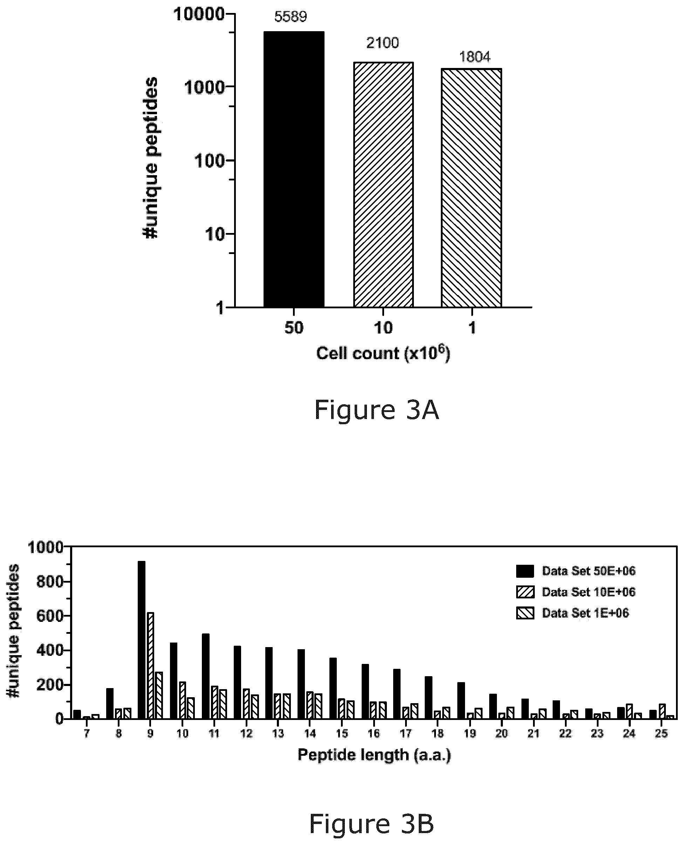

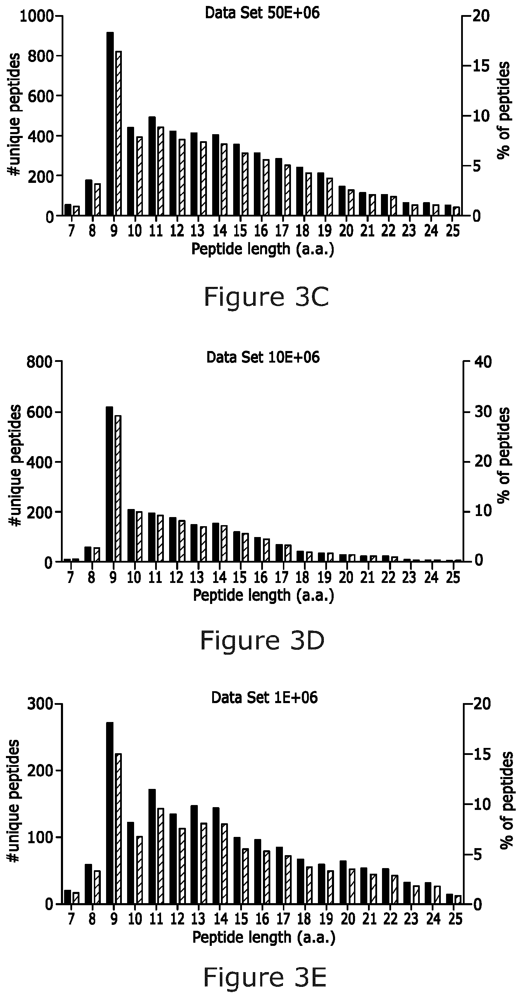

**Figure 3. Properties of the HLA-I peptidomes data set obtained from JY cell line.** A) Number of unique peptides eluted from $50\times10^6$, $10\times10^6$ and $1\times10^6$ JY cells. B) Overall peptide length distribution of the HLA peptides in the three data sets derived from the JY cell line C-E) Length distribution of HLA peptides is depicted as number of unique peptides (left y axis) and percentage of occurrence (right y axis) for $50\times10^6$ (C), $10\times10^6$ (D) and $1\times10^6$ (E).

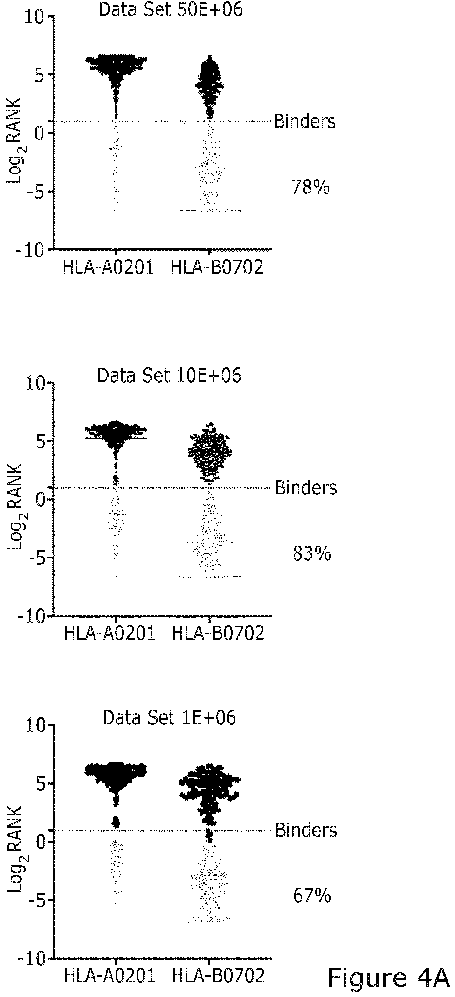

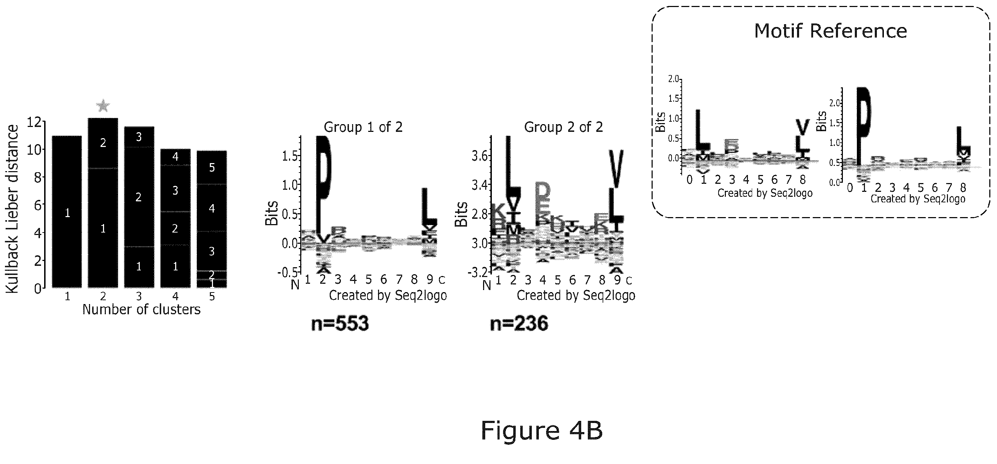

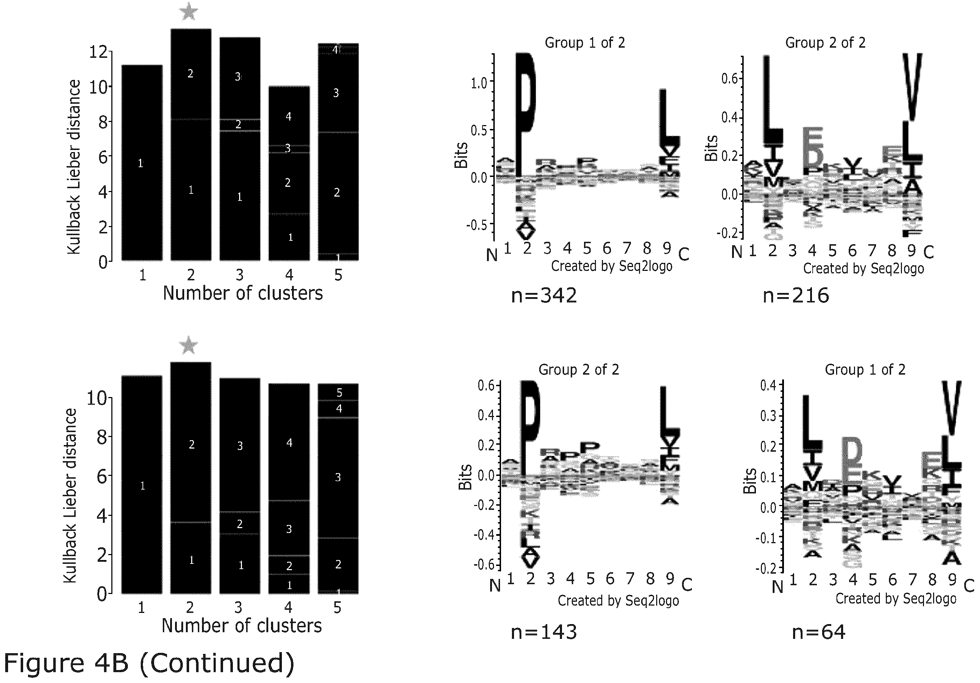

**Figure 4. Accurate analysis of HLA ligands isolated from JY cell line.** A) The eluted 9mers were analyzed as regard to their binding affinity to HLA-A*02:01 and HLA-B*07:02. The binders (green dots) and not binders (black dots) were defined in NetMHCpan 4.0 Server (applied rank 2%). B) HLA-I consensus binding motifs. Gibbs clustering analysis was performed to define the consensus binding motifs among the eluted 9mers peptides. The reference motif is depicted in the upper right corner. The clusters with the optimal fitness (higher KLD values, orange star) are showed and the sequence logo is represented with the number of HLA-I for each cluster.

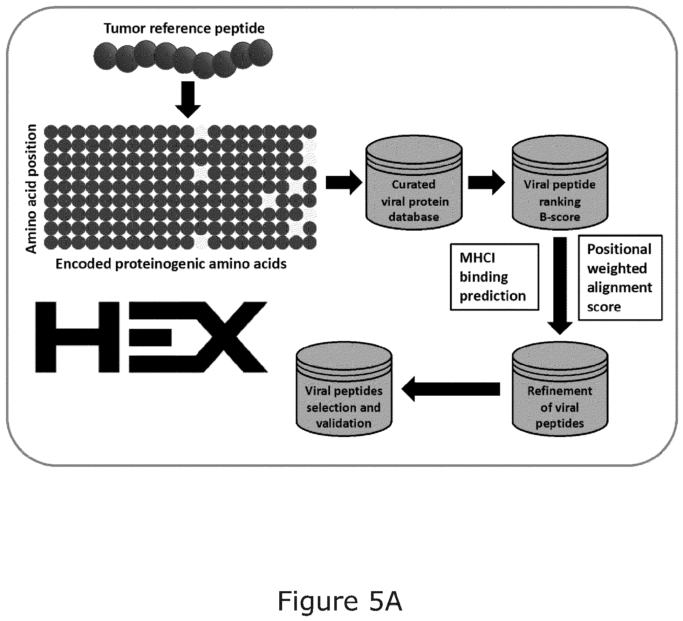

**Figure 5A. Flowchart of the HEX algorithm.** A matrix is generated based on the amino acid composition of a tumor peptide (reference peptide). This matrix is then used to scan the viral database and resulting viral peptides are ranked in order of log-likelihood of recognition. To each viral peptide is assigned an alignment score and a score for MHC-I binding prediction. The candidate viral peptides are ranked based on the following criteria: MHC-I binding prediction score > alignment score > B-score, and the highest scoring peptides are analyzed experimentally.

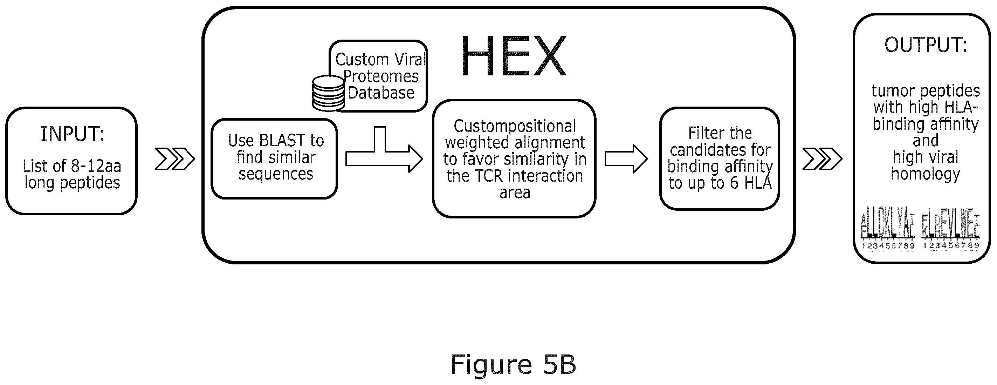

**Figure 5B. Flowcart of use of existing software.** Lists of tumor peptides of 8 to 12 amino acides in length can be used as input of the software. The tool BLAST is first employed to find the Hits (similar sequences) in the library of pathogen-derived antigens. For this task the PAM30 is generally used, however both BLOSUM and PAM substitution matrices across several evolutionary distances are supported. Next, a pairwise refinement alignment using at least BLOSUM62 and, ideally also, a substitution matrix developed by Kim et al (described in BMC Bioinformatics. 2009;10:394. Published 2009 Nov 30. doi:10.1186/1471-2105-10-394 Derivation of an amino acid similarity matrix for peptide: MHC binding and its application as a Bayesian prior. Kim Y, Sidney J, Pinilla C, Sette A, Peters B.) is performed to refine the alignment in a position-weighted fashon. Pair of peptides with high similarity in the TCR-interacting area (the central section of the peptides) receive higher similarity score. Finally, the MHC-binding affinity prediction is produced for both the tumor and the viral cognate peptide using NetMHC4.0 or NetMHCpan4.1 as standalone command line tools. The results are ultimately automatically parsed and collated within the tool and a final score which summarize the above mentioned analysis is created.

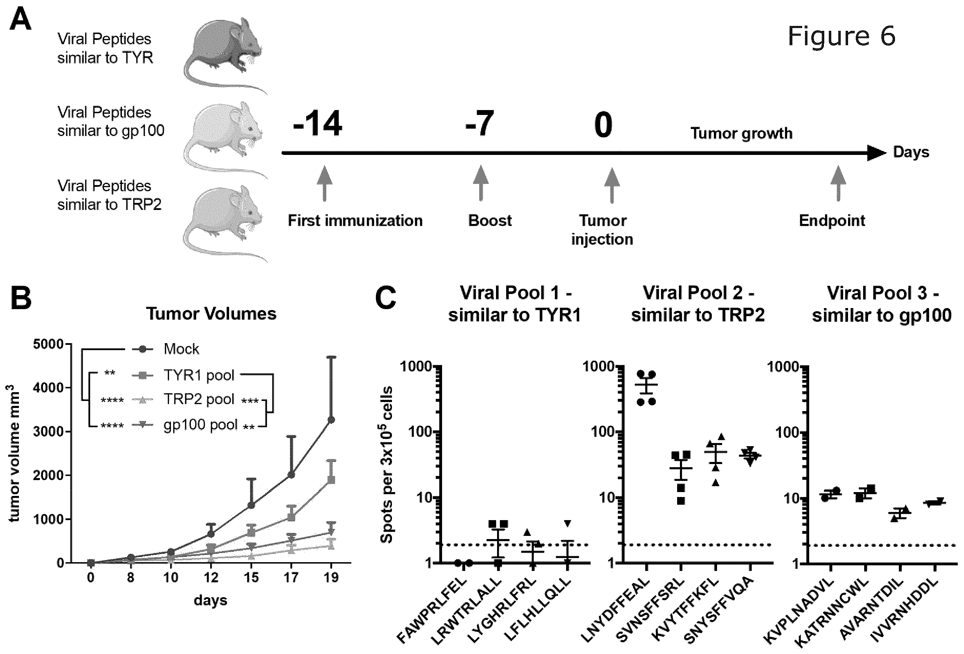

**Figure 6: Immunization with viral peptides homologous to tumor antigens slows down tumor growth.**

A: Scheme of the animal experiment: To assess if viral peptides similar to tumor peptides can impact tumor growth, 4 groups of C57BL6 mice were formed. A group of naïve mice was used as mock, the other three groups were each immunized with a different pool of viral peptides. The mice were immunized at 2 time points, 14 and 7 days, before the engraftment of the tumor.

B: After two weeks from the first immunization mice were injected subcutaneously with $3 \times 10^5$ murine melanoma B16-OVA cells. After the engraftment, tumor growth was followed by measuring with digital caliper every second day for nineteen days. P value was calculated using Two-Way ANOVA multiple comparison with Tukey's correction.

C: Mice were euthanized when the endpoint was reached. The splenocytes of the mice of each group were collected and pooled for an ELISpot assay. Each pool was then pulsed with the respective viral peptides (viral peptides homologous to TYR1, viral peptides homologous to TRP2, viral peptides homologous to GP100) to assess the response to the treatment. The dotted line shows the background produced by the negative control.

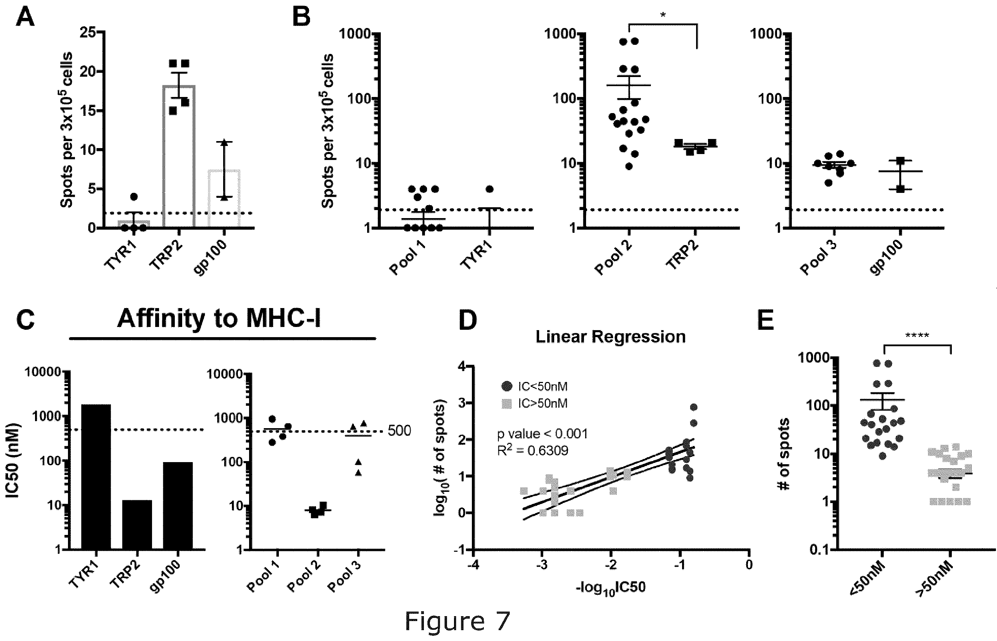

**Figure 7: Viral peptides with higher affinity for the MHC are more immunogenic and can elicit stronger cross-reactive response.**

A: To assess the response toward the respective original tumor epitope, splenocytes from each group of immunization were pooled together and pulsed with the corresponding tumor peptide.

B: Comparison between the responses elicited by pulsing the splenocytes with the pooled viral peptides and the corresponding original tumor peptide.

C: Predicted affinity of the original tumor and respective similar viral pool of peptides to the murine MHC class I. 50nM was considered as threshold to define peptides with "High-" and "Intermediate/Low Affinity" according to the IEDB guidelines.

D: Correlation between data from IFN-$\gamma$ response and predicted affinity.

E: Stratification of peptides based on their affinity and ability to stimulate IFN-$\gamma$ production. High affinity peptides (IC50 <50nM) foster significantly higher production of INF-$\gamma$ compared to Intermediate/Low affinity peptides (IC50 >50nM). P value was calculated using t test with Mann Whitney correction. The range of p value was labeled with asterisks according to the following criteria: > 0.05 (ns), $\leq$ 0.05 (*), $\leq$ 0.01 (**), $\leq$ 0.001 (***), $\leq$ 0.0001 (****).

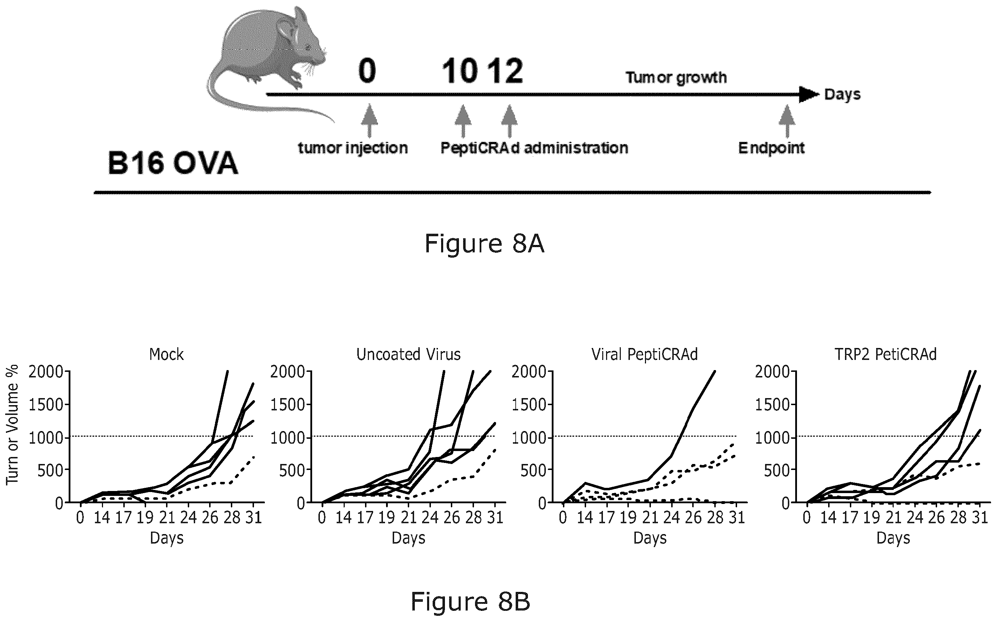

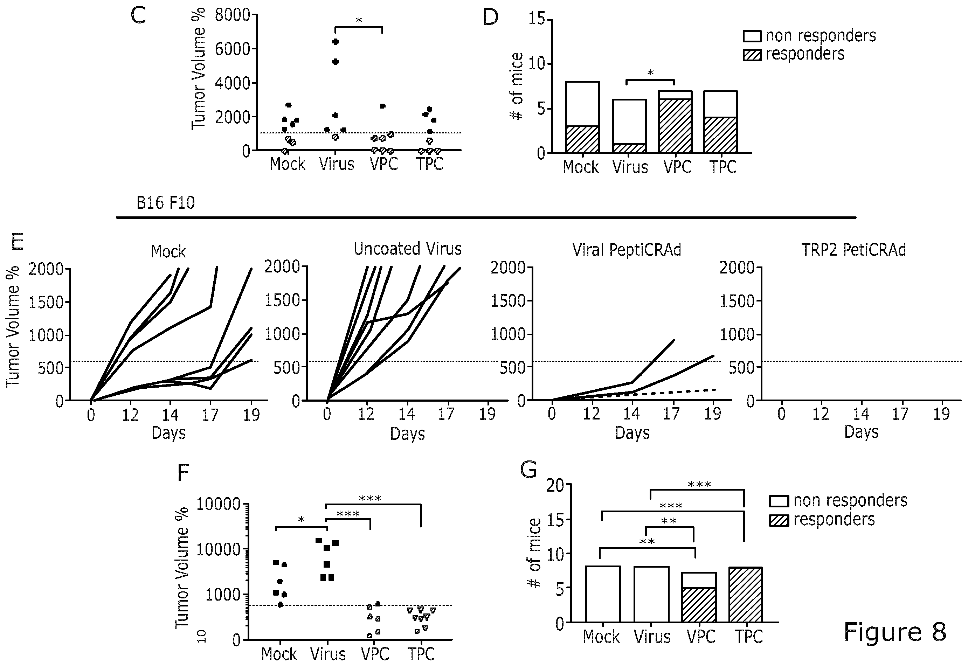

**Figure 8. Viral peptides homologous to tumor antigens can reduce tumor growth in already established tumors.**

A: Scheme of the animal experiment: 4 groups of C57BL mice were formed for each tumor cell line to be tested. Mice were subcutaneously injected either with B16-OVA or B16F10 cells at day 0. Once the tumor was palpable, mice were treated with saline solution (Mock group), uncoated adenovirus (Uncoated Virus group), adenovirus coated with pool of viral peptides homologous to TRP2 (Viral PeptiCRAd, VPC) or adenovirus coated with TRP2 peptide (TRP2 PeptiCRAd, TPC).

B: B16 OVA tumor individual growth. A threshold that defines the success of the therapy is identified by the median of all the tumor volumes of the last day.

C: B16 OVA tumor volume at the endpoint. The median of the tumor volume, showed as a dotted line, defines the success threshold of the therapy.

D: B16 OVA Contingency plot shows the number of responders per each group of treatment.

E: B16 F10 tumor individual growth. A threshold that defines the success of the therapy is identified by the median of all the tumor volumes of the last day.

F: B16 F10 Tumor volume at the endpoint. The median of the tumor volume, showed as dotted line, defines the success threshold of the therapy.

G: B16 OVA Contingency plot shows the number of responders per each group of treatment.

(C-F) P value was calculated using one-way ANOVA with Tukey's correction. The range of p value was labeled with asterisk according to the following criteria: > 0.05 (ns), $\leq$ 0.05 (*), $\leq$ 0.01 (**), $\leq$ 0.001 (***), $\leq$ 0.0001 (****).

(D-G) P value was calculated using Chi-square (and Fischer exact) test of the odds ratio. The range of p value was labeled with asterisk according to the following criteria: > 0.05 (ns), $\leq$ 0.05 (*), $\leq$ 0.01 (**), $\leq$ 0.001 (***), $\leq$ 0.0001 (****).

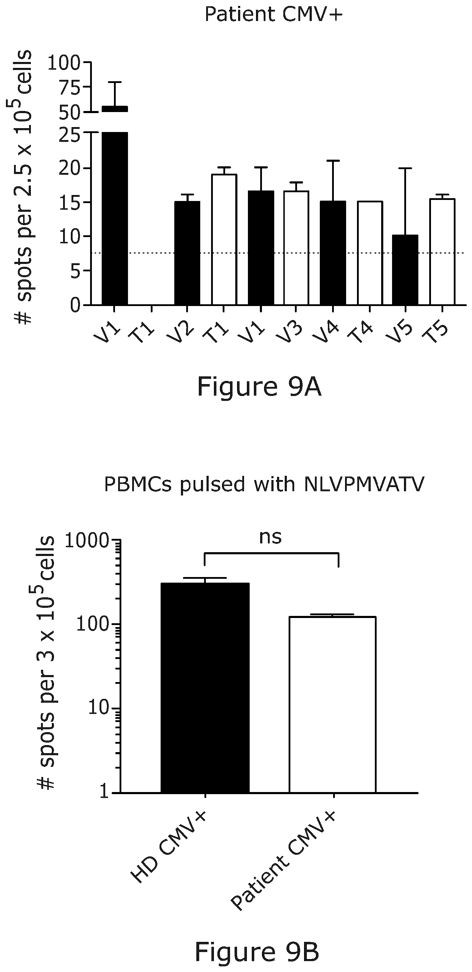

**Figure 9. T-cell cross-reactivity between viral and tumor antigens sharing high degree of homology.** PBMCs derived from patient with HLA-A*02:01 and with high serum level of anti-CMV Ab were pulsed with the peptides in Table 2. The level of IFN-$\gamma$ secreted by activated by CD8+ T-cells was detected by ELISpot assay. The dotted line indicates the noise level coming from unspecific activation of CTL in the negative control (DMSO) (A). PBMCs derived from patient with HLA-A*02:01 and with high serum level of anti-CMV Ab and from a healthy donor (HS) found positive for CMV response were tested for anti-CMV response by ELISpot assay using CMV-specific HLA-A*02:01restricted peptide NLVPMVATV. P value was calculated using t-test with Mann-Whitney correction (B).

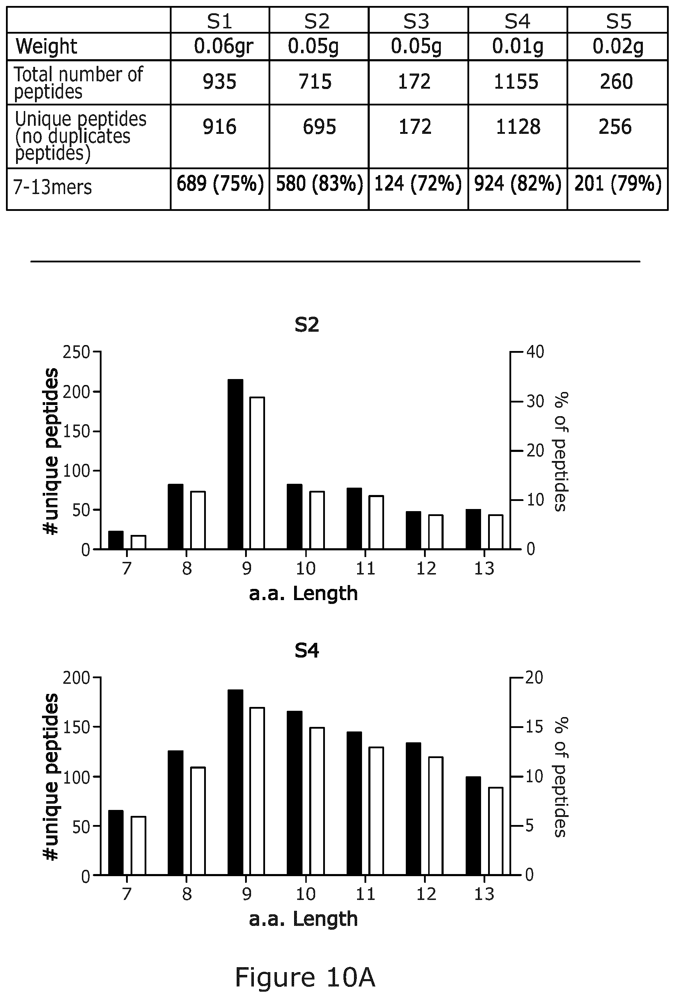

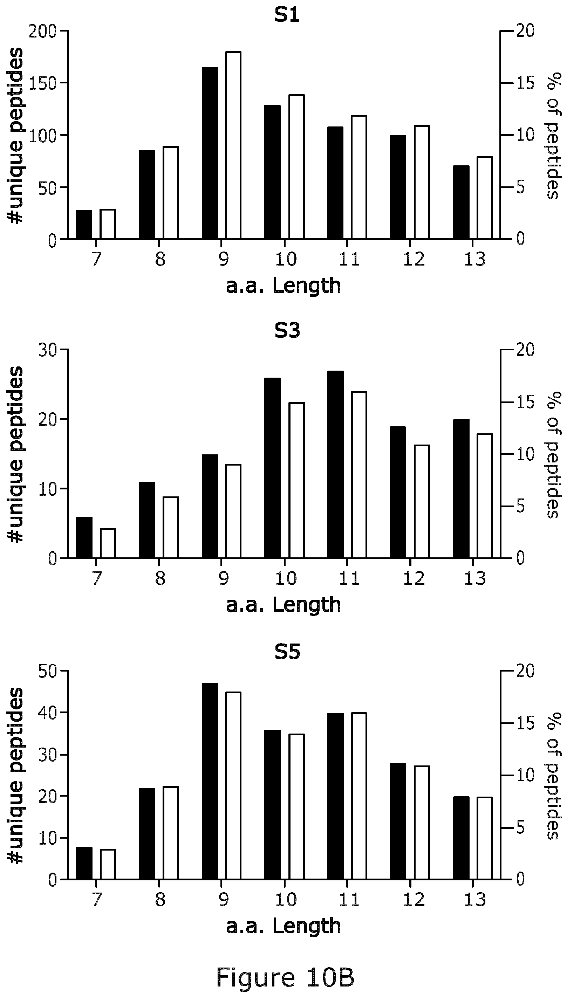

**Figure 10. Microchip based platform reveals the immunopepetidomic profile in scarce tumour biopsies.** A) The weight of the samples before the processing, the total number and the unique peptides and the enrichment in 7-13mers specimens are summarized here. B) The length distribution of the peptides as regard their absolute number and the percentage are showed as bars plot.

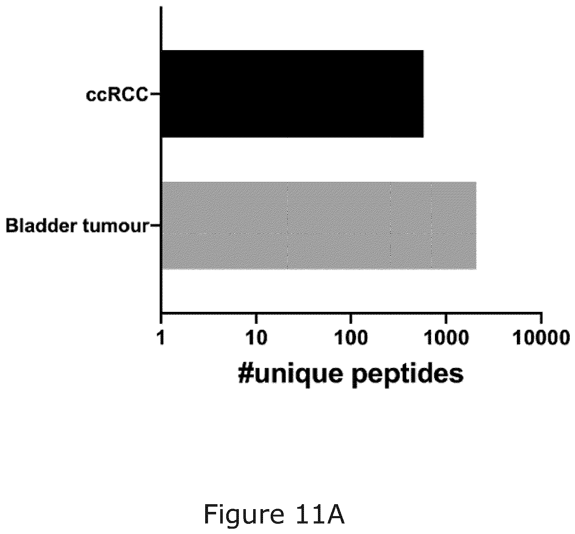

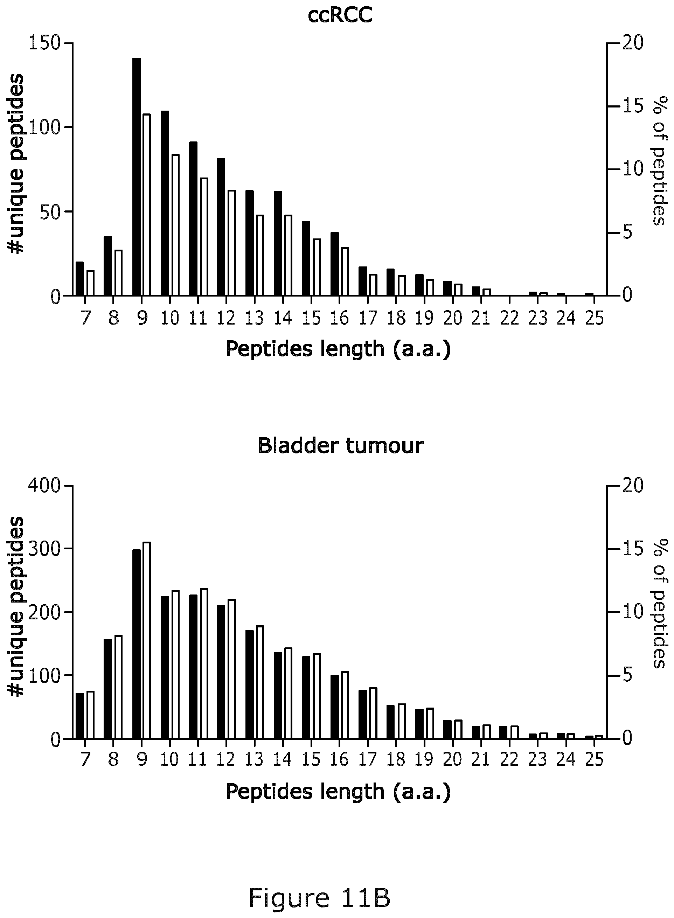

**Figure 11. Immunopeptidomic analysis of ccRCC and Bladder tumour patient derived organoids (PDO).** A) Number of unique peptides detected in ccRCC and Bladder PDOs. B) The peptides length distribution is showed as total number of unique peptides (left y axis) and percentage of occurrence (right y axis) per each PDO (ccRCC upper panel, bladder lower panel).

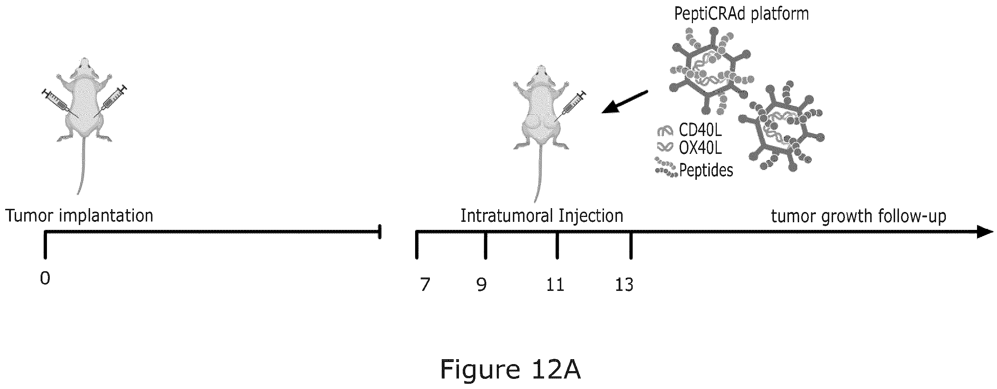

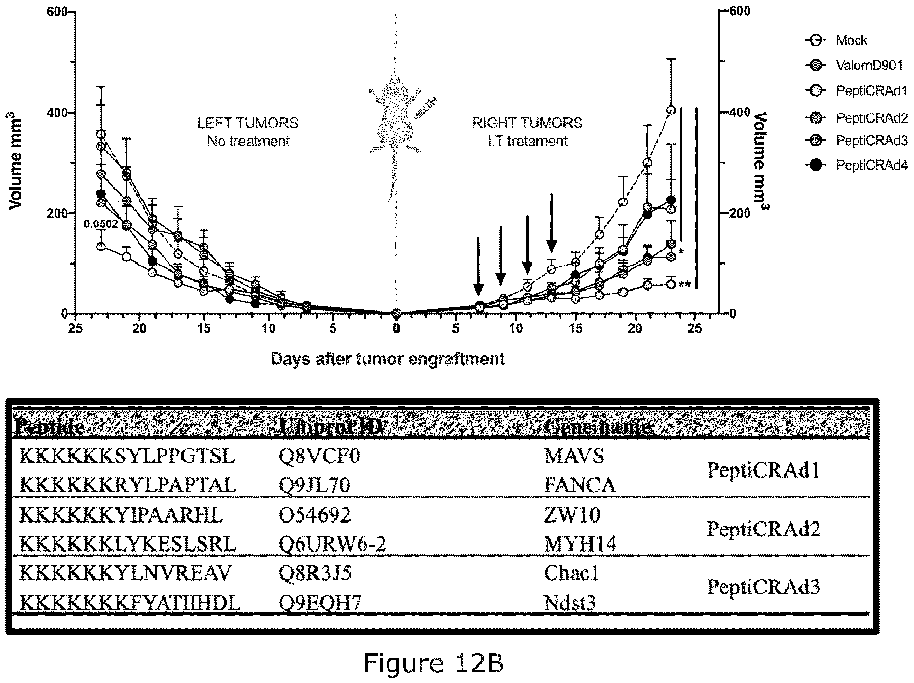

**Figure 12. Testing of the peptides.** Balb/c mice were subcutaneously injected with the syngeneic tumor model CT26 in the left and right flank. (Day 0, Fig.12A). When the tumors were established (Day 7, Fig.12A), Valo-mD901 was coated with a pair of each polylysine-modified peptide in our list (PeptiCRAd1, PeptiCRAd2, PeptiCRAd3, Table) and injected intratumorally only in the right tumor. PeptiCRAd4 consisted of Valo-mD901 coated with gp70423-431 (AH1-5), known immunodominant antigen of CT26 derived from a self-antigen encoded in the genome. Mock and Valo-mD901 groups were used as control as well. PeptiCRAd 1 and PeptiCRAd2 improved tumor growth control as well as Valo-mD901 in the injected lesions (Fig.12B, right of graph panel) as shown also in the single tumor growth per each mouse per each treatment group. Strictly, only PeptiCRAd1 significantly improved the anti-tumor activity in the untreated tumor, while Valo-mD901 triggered no effect (Fig.12B, left of graph panel).
The peptides in PeptiCRAd1 derived from HEX analysis. PeptiCRAd4 consisted of Valo-mD901 coated with gp70 423-431 (AH1-5).

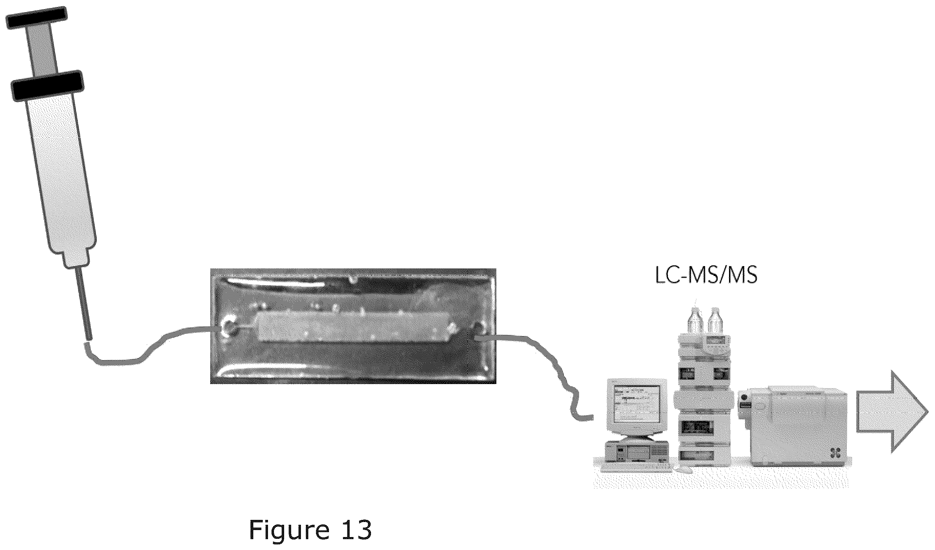

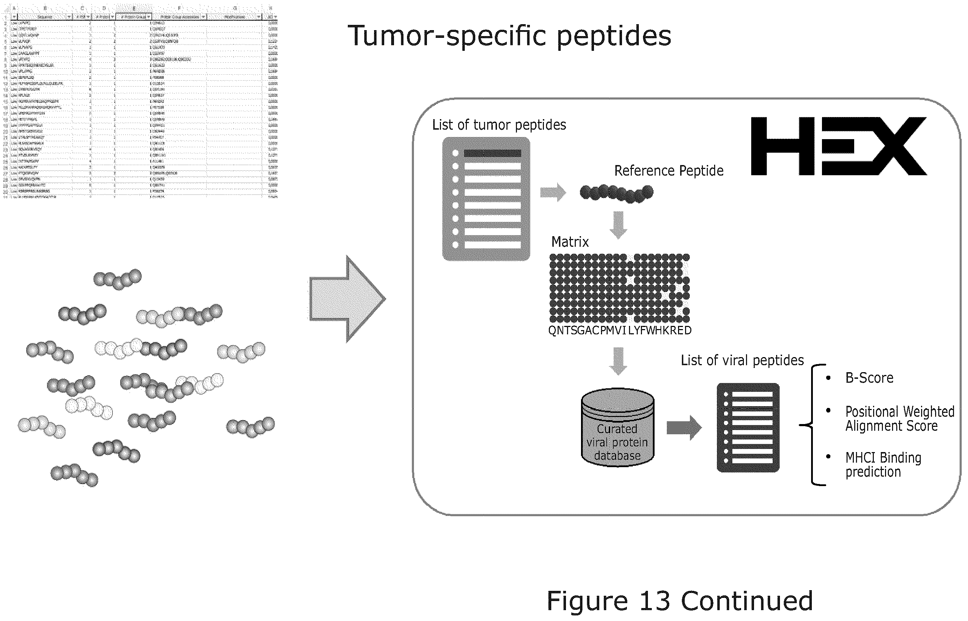

**Figure 13. A schematic overview of the invention is depicted.** The device of the invention is depicted in diagrammatic form, it has been previously functionalized with a plurality of anti-MHC (Major Histocompatibility Complex) antibodies or anti-pan-Human Leukocyte Antigen (HLA) antibodies that are able to capture pMHC (peptide: Major Histocompatibility Complex). Tumor lysate is passed trough the device and the antibodies extract pMHC. The pMHC are eluted and the peptides from the pMHC complex are analyzed, e.g. by liquid chromatography with mass spectrometry (LC-MS/MS), as a result, a list of tumor peptides is generated. Each tumour peptide is compared to a library of pathogen-derived proteins (including antigens) to determine the level of homology between each tumour peptide identifided using the device and the pathogen-derived proteins (including pathogenic antigens/pathogenic peptides) in the library. This generates a list of candidates, ideally prioritized in terms of homology, for use in cancer therapy .

**Table 1. Peptides used for the animal experiment.** Known melanoma tumor peptides (TRP2180-188, hGP10025-33 and TYR208-216) were analyzed with HEX. The best viral candidate peptides proposed by the software were selected and a pool composed by the best 4 xenopeptides per each original tumor epitope was tested *in vivo.*

**Table 2. Peptides used for the ELISpot on patients' PBMCs.** Known Melanoma-associated antigens were analyzed with HEX. The best human CMV-derived candidate peptide per each antigen was chosen to be tested *in vitro.*

**Table 3. Comparative analysis between microchip-based Immuno Precipitation technology and the standard procedure.** The table reports the total amount of antibody coated into the microchip-based IP technology and the standard procedure.

**Table 4.** Shows HEX output identified 13 tumour MHC restricted peptides with their counterpart pathogen-derived peptides.

**Table 5.** Table of peptides tested in the ELISPOT assay.

**Table 6.** Table of selected peptides for the *in vivo* PeptiCRAd assays shown in Figure 12.

**Methods and Materials**

**Device Manufacture**

The device of the invention (known as PeptiCHIP) is a flow-through construction containing thousands of pillars that are coated with a linker to which is attached the Biotin. Biotin is, in turn, connected to streptavidin which is bound with a HLA-specific capturing antibody, specifically, three molecules of HLA-specific capturing antibody.

Using conventional microfluidic manufacturing techniques, the fabrication of the PeptiCHIP is based on UV-initiated photoreaction between thiol and allyl ("ene") functional groups in according to the following off-stoichiometric ratio of 150:100 (tetrathiol: triallyl).

Immediately after the afore fabrication, the PeptiCHIP is derivatized with the biotin-PEG4-alkyne (Sigma, 764213) by UV photopolymerization followed by reaction with avidin. This is undertaken as follows.

Step 1: We prepared a 1 mM biotin-PEG4-alkyne solution (stock of biotin-PEG4-alkyne in 10 mM in ethyleglycol). Wev tooke a small aliquot and added 1% (m/v) photoinitiator (Igracure® TPO-L, BASF), using 10% photoinitiator stock in methanol, thus, the final solution is 1 mM biotin + 1% Lucirin in EG-MeOH 9:1*

We filled the chip with one volume of the solution of biotin-PEG4-alkyne and 1% Lucirin in EG:MeOH 9:1 (v/v), expose to UV for 1 min (using LED UV lamp $\lambda$=365 nm, I=15 mW/cm$^2$).

We rinsed thoroughly first with methanol, then with milli-Q water, we flushed 1-2 mL of each solvent through the channel and stored it dry (if necessary).

STEP 2: After the derivation with biotin, the chip is functionalized with streptavidin. We prepared a stock of streptavidin of 0.01 mg/ml in PBS and added it to the CHIP for 15' in the dark at room temperature. We washed 3 times with 200ul of PBS.

We added BSA 100ug/ml in 15 mM in PBS for 10' at room temperature.

STEP 3: Reaction with anti-pan-HLA antibody. We added 25 ul of biotin anti-human HLA-A,B,C 1.6 ug/ul (Biolegend CAT. Number 311434) for 15' at room temperature. We then washed three times with 200 ul of PBS and then the CHIP is ready for the immunoprecipitation of the MHC complex.

Tumor sample preparation: we detached the cells with EDTA 4mM and washed once with PBS. Add 25ul of Igepal 1% in PBS + protease inhibitors. Centrfuge 500xg 10' +4°C. Cnetrifuge 20000xg 10' +4°C.

**Optimized microfluidic pillar arrays**

Immune-purification steps were carried out within a single microfluidic chip by adding a biotinylated pan-HLA antibody to a streptavidin-prefunctionalized solid support structure (i.e., the micropillar array) and then immobilizing the HLA onto the pan-HLA antibody coated solid surface.

In summary, off-stoichiometric thiol-enes (OSTEs) polymer based micropillar arrays were fabricated with a UV-replicamolding technique and biotinylated. Next, the biotinylated micropillars were functionalized with streptavidin and the biotinylated pan-HLA antibody was added, after which the cell lysate was loaded directly into the microfluidic chip to selectively trap the HLA-I complexes. After adequate washing, the trapped HLA-I complexes were eluted at room temperature by applying 7% acetic acid (Fig. 1B). Thereafter, the protocol proceeded according to the standard immunopeptidomics workflow, including purification of the eluted HLA peptides with SepPak®-C18 in acetonitrile and evaporating them to dryness by using vacuum centrifugation.

The efficiency of the streptavidin functionalization on micropillar arrays was examined with respect to two different incubation times (15 min and 1 h), with the help of fluorescent AlexaFluor488-streptavidin. The shorter incubation time was found sufficiently long for building the first streptavidin layer (Fig. 2A). Moreover, to determine the effect of the streptavidin concentration on the final amount of immobilized biotinylated pan-HLA antibody, several concentrations of non-fluorescent streptavidin were tested in the presence of a fixed amount of the biotinylated pan-HLA antibody. In this case, the biotinylated pan-HLA antibody was incubated for 15 min followed by three washing steps with PBS (200 $\mu$l for each step). To quantitate the amount of the immobilized biotinylated pan-HLA antibody at each streptavidin concentration, a fluorescent-labelled AlexaFluor488 secondary antibody was used to titer the immobilized biotinylated pan-HLA antibody. Interestingly, even a 10-fold increase in the streptavidin concentration did not much affect the amount of immobilized biotinylated pan-HLA antibody (Fig. 2B), which likely resulted from steric hindrances limiting the number of available streptavidin binding sites. On the basis of this finding, no further concentrations of streptavidin were explored, but the highest streptavidin concentration tested (0.1 mg/mL) was used in all subsequent experiments to ensure maximal binding of the biotinylated pan-HLA antibody. However, to further investigate the selectivity of the antibody binding onto the streptavidin functionalized micropillar surface, the impact of an additional coating step with bovine serum albumin (BSA) on the amount of immobilized biotinylated pan-HLA antibody was examined with a view to eliminate nonspecific interactions. To this end, the micropillar array was preconditioned with BSA (100 $\mu$g/mL in 15mM PBS, 10 min incubation) after streptavidin functionalization and the efficiency of subsequent binding of the biotinylated pan-HLA antibody was again determined with the help of the fluorescent-labelled secondary antibody. This procedure substantially reduced the amount of immobilized pan-HLA antibody in comparison to the non-preconditioned surfaces (Fig. 2C) suggesting that nonspecific binding sites could be blocked with a simple BSA preincubation step. Therefore, the BSA incubation step was adapted for all futher experiments.

Finally, we sought to characterize the maximum amount of immobilized biotinylated pan-HLA antibody that can be bound onto a single chip by using the optimized protocol. This was evaluated through the use of multiple loading cycles of a new antibody batch of the same concentration (0.5 mg/mL) per a single microfluidic chip. In this case, the amount of the immobilized pan-HLA antibody was determined by comparing the pan-HLA antibody amount in the feed solution versus the output solution through an ELISA assay. It was observed that the amount of immobilized antibody increased almost linearly along with the number of loading cycles (Fig. 2D), allowing an accurate adjustment of the total amount of immobilized biotinylated pan-HLA antibody based on the number of loading cycles. After seven cycles, the amount of immobilized antibody reached the approximate amount of 45 $\mu$g, which suffices, at least theoretically, for the immunopeptidome investigation of scarce biological material as 10 mg of the pan-HLA ($3.88 \times 10^{16}$ molecules of antibody) is required by the state-of-the-art methodologies for the investigation of $10^9$ number of cells [22A] (Table 3).

With the microchip setup, $1.74 \times 10^{14}$ molecules of antibody could be immobilized and technically $4.5 \times 10^6$ cells could be investigated.

**Microchip-based antigen enrichment implemented in the immunopeptidomics workflow allows the identification of naturally presented HLA-I peptides**

To assess whether the developed thiol-ene microchip could be exploited as a platform for antigen discovery, we immunopurified HLA peptides from the human B-cell lymphoblastoid cell line JY. The JY line has high expression of class I HLA and is homozygous for three alleles common in the human population (HLA-A*02:01, HLA-B*07:02 and HLA-C*07:02), and it has been extensively adopted for ligandome analysis. Consequently, the JY cell line was considered a suitable model for benchmarking the microchip-based antigen enrichment Immuno Precipitation technology.

Hence, HLA-I complexes were immunoaffinity-purified using the thiol-ene microchips, functionalized with the amount of pan-HLA antibody as described above. Moreover, to determine the sensitivity of our approach, the protocol was challenged by using total cell numbers as low as $50 \times 10^6$, $10 \times 10^6$ and $1 \times 10^6$. The lysates were loaded into the microchips, and after adequate washing with PBS, the peptides were eluted with 7% acetic acid and analysed by tandem mass spectrometry. The entire workflow took an average, from the streptavidin functionalization to the elution of the tumour peptides, of <24 hours. A stringent false discovery rate threshold of 1% for peptide and protein identification was applied to generate data with high confidence. We were able to identify 5589, 2100 and 1804 unique peptides from $50 \times 10^6$, $10 \times 10^6$ and $1 \times 10^6$ cells, respectively (Fig. 3A).

As we sought to carefully analyse the ability of the microchip technology to enrich for natural HLA-I binders and to avoid potential co-eluting contaminants, we extensively characterized the eluted peptides. First, the eluted peptides from the JY cell line represented the typical length distribution of a ligandome data set, with 9mers as the most enriched peptide species (Fig. 3B-E). Next, the predicted binding affinity for the two HLA-I alleles (HLA-A*0201 and HLA-B*0702) expressed in JY cells was determined. JY cells also have a low level of the allele HLA-C*0702, but the binding motif overlaps with the motifs of HLA-A*0201 and HLA-B*0702; hence, only these alleles were considered in the subsequent analysis.

Of the unique 9mers, 78%, 83% and 67% were predicted to be binders (described as binders in NetMHCpan4.0, applied rank 2% [24A-26A]) to either HLA-A*0201 or HLA-B*0702 alleles for $50 \times 10^6$, $10 \times 10^6$ and $1 \times 10^6$ cells, respectively (Fig. 4A). Moreover, Gibbs analysis was performed to deconvolute the consensus binding motifs of respective HLA-I alleles from the eluted 9mer peptides; these clustered in two distinct groups, with a preference for reduced amino acid complexity for residues at positions P2 and Ω, matching remarkably well with the known ones for HLA-A*0201 and HLA-B*0702 (Fig. 4B).

Next, in order to determine the role of the peptides identified, a gene ontology (GO) term enrichment analysis was performed on our list of 9mer binder source proteins. We observed an enrichment in nuclear and intracellular proteins, mainly those interacting with DNA, RNA or involved in catabolic activity. Finally, we set up an *in vitro* killing assay to further demonstrate the capacity of the microchip technology in isolating peptides in complex with HLA-I. To this end, a set of three peptides was selected from our JY data set to stimulate HLA-matched PBMCs; CD8+ T cells were purified from the PBMCs and adopted as effector cells in coculture with JY cells. To account for nonspecific cytotoxicity due to the effector cells per se, unstimulated PBMCs were used as a control. Real time cytolysis was then monitored. Interestingly, the CD8+ T cells pulsed with the peptides QLVDIIEKV (SEQ ID NO: 75; gene name PSME3) and KVLEYVIKV (SEQ ID NO: 76; gene name MAGEA1) showed about 10% specific cytolysis, whereas the CD8+ T cells pulsed with the peptide ILDKKVEKV (SEQ ID NO: 77; gene name HSP90AB3P) induced 15% specific cytolysis, indicating specific lysis in the presence of defined peptides.

To evaluate the validity of our HLA-I peptide lists identified by the microchip technology, we interrogated SysteMHC, a repository of the immunopetidomics data set generated by mass spectrometry. Among the unique 9mer binders identified in our data, 69%, 77% and 81% were also found in a previously published ligandome data set derived from the JY cell line (pride ID PXD000394) [3A] for $50 \times 10^6$, $10 \times 10^6$ and $1 \times 10^6$ cells, respectively (Fig. 6A). In addition, a positive correlation between the abundance of the source protein and HLA presentation was confirmed, with the most abundant proteins being the main source of the HLA peptides.

Hence, these results demonstrated that the chip-based protocol can be exploited as a reliable Immuno Precipiation platform within the immunopeptidomic workflow.

## Device Use

Application of the sample to chip and elution of fractions for later analysis

We applied the sample through multiple cycles and incubated for 5 minutes (working at 4°C). We washed 3 times with 200ul of PBS and aspirated the last washing to empty the chip. We prepared a solution of 50%MeOH and 50%MilliQ. With the previous solution we prepared a 7% solution of Acetic Acid. We applied this solution to the CHIP and collected the fraction. The elution time was 5 minutes. On the same day, we purified the collected fractions. We prepared a SepPak cartridge for each tissue sample-for the HLA-I peptides sample and labelled them. Using a syringe and the dedicated adaptor we washed the cartridge first once with 1ml of 80%ACN in 0.1%TFA, then twice with 1ml of 0.1% TFA. We load each of the biological samples on a SepPak cartridge. We passed them through slowly (speed of about 1ml in 20s). We washed the cartridges twice with1 ml of 0.1%TFA. We eluted the HLA binding peptides into a collection tube with 300ul of 30% ACN in 0.1%TFA.

## HEX (Homology Evaluation of Xenopeptides)

Homology Evaluation of Xenopeptides (HEX) is a novel *in silico* platform that compares similarity between tumor peptides (reference peptides) and pathogen-derived, such as viral, peptides (query peptides). It utilizes several metrics in order to expedite candidate peptide selection. This is done by incorporating both novel methods (peptide scoring and alignment scoring algorithm) and integrated pre-existing methods (MHC-I binding prediction). HEX comes with a number of precompiled databases of known proteins, such as proteins derived from viral pathogens and the human proteome (33).

The associated scoring matrix is generated *ad hoc* based on the amino acid composition of the reference peptide, as opposed to experimentally. In particular, in the matrix rows representing the amino acid position in the peptide and columns representing each of the 20 standard amino acids, amino acid positions of the reference peptide were assigned the same high score and other positions were assigned the same low score.

Alignments are computed pairwise between peptides in the query set against the reference set. For a given pair of peptides, their alignment is calculated by the summing the distance scores between pairs of amino acids in the same position. Scoring is weighted to prioritize similarity between more central amino acids in the peptide. HEX supports both BLOSUM and PAM substitution matrices across several evolutionary distances. Specifically, BLOSUM 62 was the matrix chosen for this study.

MHC class I binding affinity predictions are made using NetMHC [27] via the Application programming interface of IEDB (http://tools.iedb.org/main/tools-api/) and are then parsed and collated within the tool. The user can specify their desired scoring method or return a number of recommended results. Predictions for a number of human and murine MHC-I alleles are supported.

Users are able to select peptides by their own criteria or allow peptides to be selected by a random forest model. The random forest was trained on experimental outcomes of peptides chosen by the authors. Feature importance was determined by out-of-bag (OOB) increase in mean squared error (MSE) and cross-validated on an unseen sample of the peptides. HEX was developed as a web application using the R package Shiny and is accessible at https://picpl.arcca.cf. ac.uk/hex/app/ without user registration. The source code is available at https://github.com/whalleyt/hex.

In an alternative embodiment of the invention, HEX supports both BLOSUM and PAM substitution matrices across several evolutionary distances. Specifically, BLOSUM 62 was the matrix used in this study. HEX, first performs a BLAST search using PAM30 to find the Hits (similar sequences) in the reference library. Next, HEX performs a pairwise refinement alignment using both BLOSUM62 and a substitution matrix developed by Kim et al.

Ref: Kim Y, Sidney J, Pinilla C, Sette A, Peters B. Derivation of an amino acid similarity matrix for peptide: MHC binding and its application as a Bayesian prior. BMC Bioinformatics. 2009;10:394. Published 2009 Nov 30. doi:10.1186/1471-2105-10-394.

MHC class I binding affinity predictions are made using NetMHC4.0 (or NetMHCpan4.1) as a standalone command line tool and are then parsed and collated within the tool. The user can specify their desired scoring method or return a number of recommended results. Predictions for a number of human and murine MHC-I alleles are supported.

**Patients and samples**

In total, 16 stage 4 metastatic melanoma patients were treated with anti-PD1 monoclonal antibody in the Helsinki University Central Hospital (HUCH) Comprehensive Cancer Center. Patients were randomly selected to receive either nivolumab (n=7) infusions every second week or pembrolizumab (n=9) infusions every third week. The study was approved by Helsinki University Central Hospital (HUCH) ethical committee (Dnro 115/13/03/02/15). Written informed consent was received from all patients and the study was conducted in accordance with the Declaration of Helsinki.

Peripheral blood samples (3ml EDTA blood, 50ml Heparin blood) were collected from three time-points; before initiation of treatment, after one and three months of treatment. From these the plasma was separated by centrifuging and then stored at -70°C. The CMV and EBV IgG levels were measured from thawed EDTA plasma samples using VIDAS CMV IgG (BioMérieux, Marcy-I'Etoile, France) and Siemens Enzygnost Anti-EBV/IgG kits (Siemens Healthcare Diagnostics, Marburg, Germany). The immunoglobulins (IgA, IgM, IgG) from thawed Heparin plasma were measured in the central laboratory of the Helsinki University Central Hospital (HUSLAB).

**Cell lines and human samples**

The murine melanoma cell line B16-F10 was purchased from the American Type Culture Collection (ATCC; Manassas, VA, USA). Cells were cultured in RPMI (Gibco, Thermo Fisher Scientific, US) with 10% fetal bovine serum (FBS) (Life Technologies), 1% Glutamax (Gibco, Thermo Fisher Scientific, US), and 1% Penicillin and Streptomycin (Gibco, Thermo Fisher Scientific, US) at 37°C/ 5% $CO_2$.

The cell line B16-OVA, a mouse melanoma cell line modified to constitutively express chicken Ovalbumin (OVA), was kindly provided by Prof. Richard Vile (Mayo Clinic, Rochester, MN, USA). These cells were cultured in RPMI Low glucose (Gibco, Thermo Fisher Scientific, US) with 10% FBS (Gibco, Thermo Fisher Scientific, US), 1% Glutamax, 1% Penicillin and Streptomycin (Gibco, Thermo Fisher Scientific, US) and 1% Geneticin (Gibco, Thermo Fisher Scientific, US) at 37°C/ 5% $CO^2$.

All cells were tested for mycoplasma contamination with a commercial detection kit (Lonza - Basel, Switzerland). Isolated human PBMCs were frozen in FBS supplemented with 10% DMSO and then maintained in liquid nitrogen until use. Cryopreserved PBMCs were thawed and rested overnight at 37°C/ 5% $CO^2$ in complete RPMI medium supplemented with 10% FBS, 1% Glutamax, 1% penicillin-streptomycin over night before plating them for ELISPOT.

**Peptides**

All the peptides used in this study were purchased from Zhejiang Ontores Biotechnologies Co. (Zhejiang, China) 5mg >90% purity. The sequences of all the peptides used in this study are found in Table 1 and 2.

**PeptiCRAd preparation**

All PeptiCRAd complexes described in this work were prepared by mixing Adenoviruses and polyK-tailed peptides according to the following protocol: $1 \times 10^9$ vp(viral particles) were mixed with 20ug poly-K tailed peptides (resuspended in water); after vortexing, the mixture was incubated at room temperature for 15 min; PBS was added, after the incubation, up to the injection volume (50uL/mouse), successively, the solution was vortexed again and used for assays or animal injections. For the TRP2-PeptiCRAd, $1 \times 10^9$ vp were mixed with 20ug of 6K-TRP2$_{180\text{-}188}$ peptide, while the Viral-PeptiCRAd was prepared using $1 \times 10^9$ vp mixed with 5ug of each viral 6K-peptide homologous for TRP2$_{180\text{-}188}$.

New PeptiCRAds were prepared before each experiment using fresh reagents. All dilutions of virus and peptides required before incubation for PeptiCRAd preparation, were performed in sterile PBS or water. The PeptiCRAds were then diluted in the buffer required by the assay.

Viruses were generated, propagated, and characterized as elsewhere described [20].

**Animal experiments and ethical permits**

All animal experiments were reviewed and approved by the Experimental Animal Committee of the University of Helsinki and the Provincial Government of Southern Finland.

All the experiments were carried using C57BL/6JOlaHsd mice obtained from Scanbur (Karlslunde, Denmark).

For the immunization experiment 8- to 9-week-old immune competent female C57BL/6J mice were divided in 4 groups. N = 3 mice were used as mock group, n = 7 mice were used to form each of the three different treatment groups. Each treatment group was vaccinated with a different group of xenopeptides. Mice were vaccinated twice and injections were performed at one-week interval (day 0 & 7) at the base of the tail with 40ug of peptides and 40ug of adjuvant (VacciGrade poly(I:C) - Invivogen) in a final injectable volume of 100ul. Naïve mice (PBS injected) were used as Mock group. At day fourteen mice were injected with $3 \times 10^5$ B16-OVA cells on the right flank and tumor growth was followed until endpoint was reached.

For the treatment of established tumors, we tested 2 different tumor cell lines: B16-OVA cells and the more aggressive B16F10 cells. $3 \times 10^5$ B16-OVA cells and $1 \times 10^5$ B16-F10 were injected subcutaneously on the right flank of 8- to 9-week-old immune competent female C57BL/6J mice. Successively, these mice were randomly divided in 4 groups of 7-8 mice for each tumor cell line. A mock group was treated with PBS; a second group was treated with uncoated Adenovirus; a third group was treated with Adenovirus coated with TRP2$_{180\text{-}188}$ (TRP2-PeptiCRAd); the last group was treated with Adenovirus coated with TRP2-homologous viral peptides (Viral-PeptiCRAd).

Mice were intratumorally treated twice and injections were performed at two days interval (day 10 & 12 from tumor engraftment) and tumor growth was followed until endpoint was reached. The median of the tumor volume measurement of the last day identifies the therapeutic success threshold showed as a dotted line. Mice that at the end point showed a tumor volume below the threshold were considered responders, while mice above it were considered as non-responders.

Tumor growth was followed with a digital caliper measuring two dimensions of the tumor. Successively, the volume was mathematically calculated according to the following formula:

$$( (long\ side) \times (short\ side)^2 )/2$$

In all experiments, tumors were measured every second or third day until the tumor size reached the maximum allowed, and mice were then sacrificed and spleens collected.

**ELISpot Assay**

To assess the amount of active antigen specific T-cells, interferon-$\gamma$ (IFN-$\gamma$) secretion was measured by ELISPOT assay from IMMUNOSPOT (CTL, Ohio USA) for the murine IFN-$\gamma$ and MABTECH (Mabtech AB, Nacka Strand, Sweden) for the human IFN-$\gamma$.

Fresh mice splenocytes collected at the end point of the experiment have been used. The procedure was carried out according to the manufacturer instructions. In brief, for murine IFN-$\gamma$, $3 \times 10^5$ of splenocytes/well were plated at day 0. Cells were stimulated with 2ug/well of peptides. After 3 days of incubation at 37°C/ 5% CO2, plates were developed according to the kit's protocol.

For human IFN-γ ELISPOT, human PBMCs were thawed and let rest over-night at 37°C/ 5% $CO_2$ in complete medium. The following day, $3x10^5$ PBMCs/well were plated and stimulated with 2ug/well of peptides. After 48h of incubation at 37°C/ 5% $CO_2$ the plates were developed according to the manufacturer's protocol. Plates were sent to CTL-Europe GmbH to be analyzed.

## Cell line and reagents

The EBV-transformed human lymphoblastoid B-cell line JY (ECACC HLA-type collection, Sigma Aldrich) was cultured in RPMI 1640 (GIBCO, Invitrogen, Carlsbad, CA, USA) supplemented with 1% GlutaMAX (GIBCO, Invitrogen, Carlsbad, CA, USA) and 10% heat inactivated foetal bovine serum (HI-FBS, GIBCO, Invitrogen, Carlsbad, CA, USA).

Streptavidin (Streptomyces avidinii, affinity purified, lyophilized from 10 mm potassium phosphate, ≥13 U/mg protein) was purchased from Sigma-Aldrich (Saint Louis, Missouri, USA).

Biotin-conjugated anti-human HLA-A, B, C clone w6/32 was purchased from Biolegend (San Diego, CA, USA) for analysis.

The following peptides were purchased from Ontores Biotechnologies Co., Ltd., were used throughout the study: KVLEYVIKV (SEQ ID NO: 75; gene name MAGE A1), ILDKKVEKV (SEQ ID NO: 76; gene name HSP90), and QLVDIIEKV (SEQ ID NO: 77; gene name PSME3).

Additionally, the following peptides were purchased from Chempeptide (Shangai, China): VIMDALKSSY (SEQ ID NO: 78; gene name NNMT), FLAEGGGVR (SEQ ID NO: 79; gene name FGA) and EVAQPGPSNR (SEQ ID NO: 80; gene name HSPG2).

## Ovarian tumour biopsy and ethical consideration

The ovarian tumour biopsy was collected from a patient with ovarian metastatic tumour (high grade serous), who signed an informed consent, under the studies approved by the Research Ethics Committee of the Northern Savo Hospital District with the approval number 350/2020. The samples were chopped in small pieces and treated with a digestion buffer containing collagenase type D (Roche) 1mg/ml, Hyaluronidase (Sigma Aldrich) 100 μg/ml and DNase I (Roche) 1mg/ml for 1h at 37°C. The cell suspension was sequentially passed through a 500 μm and 300 μm cell strainer (pluriSelect) to obtain single cells.

## Renal cell carcinoma and bladder tumour samples and ethical considerations

Patient tissue samples for organoid cultures were obtained from the DEDUCER study (Development of Diagnostics and Treatment of Urological Cancers) at Helsinki University Central Hospital with approval number HUS/71/2017, 26.04.2017, ethical committee approval number 15.03.2017 Dnro 154/13/03/02/2016, and patient consent. The kidney sample was obtained from a nephrectomy of an adult male with a clear cell renal cell carcinoma (ccRCC, pTNM stage pT3a G2). The benign kidney tissue sample was used for the experiments. The carcinoma urothelial (bladder cancer, high grade, gradus III, 1x1 cm) was obtained from adult female, and the cancer tissue sample was used for the organoid culture.

## Clear cell renal carcinoma and bladder tumour organoid culture

Cells were isolated from the original tissue instantly after surgery by dissociating the tissue into small pieces and treating it with collagenase (40 units/ml) for 2-4 h. Benign and cancer cells of the kidney of a clear cell renal cell carcinoma patient cells were grown as organoids in F-medium [3:1 (v/v) of F-12 nutrient mixture (Ham) - DMEM (Invitrogen), 5% FBS, 8.4 ng/mL cholera toxin (Sigma), 0.4 μg/mL hydrocortisone (Sigma), 10 ng/mL epidermal growth factor (Corning), 24 μg/mL adenine (Sigma), 5 μg/mL insulin (Sigma), 10 μM ROCK inhibitor (Y-27632, Enzo Life Sciences, Lausen, Switzerland) and 1% penicillin-streptomycin with 10% Matrigel (Corning). The bladder tumour-derived organoids were grown in hepatocyte calcium medium (Corning)[15A] supplemented with 5% CSFBS (Thermo Fisher Scientific), 10 μM Y-27632 RHO inhibitor (Sigma), 10 ng/mL epidermal growth factor (Corning), 1% GlutaMAX (Gibco), 1% penicillin-streptomycin and 10% Matrigel. $6x10^6$ cells were collected by centrifugation, washed in PBS to remove Matrigel and snap frozen before analyses.

## HLA typing

The clinical HLA typing of tumour samples (ccRCC and bladder) was performed by the European Federation for Immunogenetics (EFI) -accredited HLA laboratory of the Finnish Red Cross Blood Service. Allele determination of three classical HLA-I genes HLA-A, -B and -C was performed by targeted PCR based next generation sequence (NGS) technique according to the protocol provided by the manufacturer (NGSgo® Workflow, GenDx, Utrecht, The Netherlands).

The allele assignment at 4-field resolution level was implemented by NGSengine Version: 2.11.0.11444 (GenDx, Utrecht, The Netherlands) using IPD IMGT/HLA database Release 3.33.0, https://www.ebi.ac.uk/ipd/imgt/hla/.

**Flow cytometry analysis**

The following antibodies were used to analyse the cell surface expression of HLA-A2 and HLA-A, B, and C: PE-conjugated anti-human HLA-A2 (clone BB7.2, BioLegend 343306 San Diego, CA, USA), PE-conjugated anti-human HLA-A, B, and C (clone W6/32, BioLegend 311406, San Diego, CA, USA), and Human TruStain FcX block (BioLegend B247182, San Diego, CA, USA).

The data were acquired using a BDLSR Fortessa Flow Cytometer. Flow cytometric analysis of renal cell carcinoma and bladder tumour-derived organoids was performed using a BD Accuri 6 plus (BD Biosciences) and analysed with FlowJo software (Tree Star, Ashland, OR, USA).

**Results**

*Development of Homologous Evaluation Xenopeptides (HEX) tool for identification of viral- and tumor-derived peptides with high molecular mimicry*

In order to study whether molecular mimicry between viruses and tumor could impact on tumor growth we needed to identify peptides sharing a high degree of homology. However, a tool to facilitate the identification of relevant target, to this scope, is lacking. Therefore, we developed HEX (Homology Evaluation of Xenopeptides), a device that compares the input sequences to a database of pathogen-derived antigens/peptides or protein sequences and selects highly homologous candidate pairs of peptides based on at least two of the three following criteria: 1) A *B-score* that corresponds to the likelihood of the peptides being recognized by a given TCR; 2) A *positional weighted alignment score* to prioritize the similarity in the area of interaction with the TCR; 3) the prediction of the *MHC class I binding affinity* (Figure 5A). In the alternative the use of conventional software may be used as shown in Figure 5B and the as described above.

We started from three melanoma-associated antigens, that have been applied successfully in a number of vaccination studies: TRP2$_{180-188}$ (*tyrosinase-related protein* 2), GP100$_{25-33}$ (aka PMEL; *premelanosome protein)* and TYR1$_{208-216}$ (*tyrosinase 1).*

TYR1$_{208-216}$ is not predicted to be a binder of the murine MHC, and thus, was considered as an *"irrelevant target".* With HEX we identified viral peptides that shared a high degree of homology with the input tumor epitopes. Pools of 4 viral-derived peptides per each original tumor epitope (Table 1) were chosen to be further evaluated *in vivo.*

*Anti-viral immunity controls tumor growth via molecular mimicry with tumor antigens*

To assess whether the viral-derived peptides would influence the tumor growth, we decided to mimic the viral infection by immunizing C57BL6 mice with selected viral peptide-pools followed by the tumor engraftment (Figure 6A). All immunized mice showed a reduction in tumor growth compared to mock. Additionally, significant differences between the treatment groups were observed. The tumor growth was most reduced in mice immunized with viral-pool of peptides homologous to TRP2 or gp100 (Figure 6B).

To further investigate the contribution of the selected viral peptides in the reduction of tumor growth, we collected the splenocytes of the mice at the endpoint for ELISpot assay (Figure 6C). The splenocytes from mice immunized with viral peptide-pools homologous to TRP2 or gp100 fostered higher IFN-$\gamma$ response compared to the splenocytes from mice immunized with control TYR1-homologous epitopes, in correlation with the outcome of the tumor growth.

We further investigated the reactivity of these splenocytes from the viral pre-immunized mice towards their respective cognate tumor antigen (Figure 7A). When compared side by side only the viral peptides homologous to TRP2 showed higher IFN-$\gamma$ secretion compared to the cognate tumor peptide (Figure 7B). In addition, we retrospectively assessed the affinities of each tumor antigen and their homologous viral pools to the C57BL/6J MHC class I molecules (Figure 7C) and observed significant correlation with their respective ability to stimulate IFN-$\gamma$ secretion (Figure 7D). Following the guidelines of the Immune Epitope Database (IEDB) we divided the peptides as High and Low affinity using the threshold of 50nM (http://tools.iedb.org/mhci/help/) and observed that peptides with high MHC-I affinity fostered the production of more INF-$\gamma$ compared to peptides with low MHC-I affinity (Figure 7E). Taken together these results validate the predictive efficiency of HEX tool to identify homologous peptides, and highlight that molecular mimicry between viruses and tumor plays a role in anti-tumor immune response through cross-reactive T-cells.

*Viral epitopes sharing high similarity to tumor epitopes reduces the growth of established melanoma in vivo*

We have shown that the molecular mimicry between viral and tumor antigens can affect the tumor growth in preimmunized mice. Next, we wanted to assess whether the molecular mimicry directed-response could have an impact on already established tumors in naïve mice. To this end, mice were implanted with B16OVA tumors, or more aggressive and immunosuppressive B16F10 tumors and, subsequently, treated with the previously developed vaccine platform PeptiCRAd (20) to mimic a viral infection (Figure 8A). Briefly, PeptiCRAd is a vaccinal technology consisting of adenoviruses coated with MHC-I restricted peptides bearing positively charged poly-aminoacidic tales. In this case, we proceeded using the most effective pool of peptides from the first *in vivo* experiment. Vaccines were prepared by coating adenoviruses with the original $TRP2_{180-188}$ epitope (TRP2-PeptiCRAd) or the corresponding pool of viral-derived peptides similar to TRP2 (Viral PeptiCRAd). Intratumoral injections of PeptiCRAd significantly reduced the tumor progression compared to treatment with saline buffer or uncoated-virus both for B16OVA (Figure 8B-C) and B16F10 tumors (Figure 8E-F). Mice treated with the original tumor antigen or the viral homologous showed significantly higher number of responders in both of the tumor models (Figure 8D, 8G). In both tumor models we observed a significant reduction in tumor growth when mice were treated with viral peptides similar to tumor peptides, suggesting once again that molecular mimicry might play a fundamental role in anti-tumor immunity engaging cross-reactive T cells.

*Study whether molecular mimicry between CMV and tumor antigens could explain the better prognosis*

We selected a pool of melanoma-associated proteins [21] that were compared to the CMV proteome using HEX generating a list of tumor peptides with high similarity to CMV (Table 2). Tumor peptides and their CMV counterpart were used in ELISPOT assay showing that in a responder patient, PBMCs always responded to both viral and their counterpart tumor antigens, suggesting that the CMV infection had expanded viral T cell clones that could attack and kill tumor cells making seropositive patients more prone to react towards melanoma specific epitopes similar to CMV (Figure 9A, B). Although tantalizing, this observation suffered the limitation that did not directly show if viral and tumor peptides had expanded the same T cell clones.

### The novel microfluidic chip-based platform identifies the immunopeptidome profile in scarce tumour biopsy tissue

We challenged the platform for the investigation of a scarce tumour biopsy. Thus, an ovarian metastatic tumour (high grade serous) was collected from the patient and four pieces were derived from the tumoral border (S1, S2, S3 and S4); the central part of the tumour was collected as well (S5). Next, the samples were weighed and as summarized in Fig. 10A, the size averaged from a 0.01g to 0.06g. After the sample digestion, the obtained single cell suspensions were lysed and processed through the microchip. Applying a stringent false discovery rate threshold of 1% for peptide and protein identification, 916, 695, 172, 1128 and 256 unique peptides were identified respectively in S1, S2, S3, S4 and S5 (Fig. 10A). In line with a typical ligandome profile, a general enrichment (above 70%) was observed in 7-13mers specimens (Fig.10A). As regard absolute number and percentage, the aminoacidic length distribution showed that the 9mers specimens were the most representative (Fig.10B), confirming our and other previous immunopeptidomic analysis. Next, we further investigated the source proteins found in our data, applying Gene Ontology (GO) enrichment analysis. Consistently with a typical ligandome profile, metabolic processes were enriched in all the samples examined. Additionally, the analysis revealed an increase in skin development pathway proteins in line with the epithelial nature of the ovarian serous tumour here analyzed. Altogether, the results highlighted the feasibility of exploiting the developed microfluidic-chip platform to analyse scarce tumour biopsy and obtain personalized antigenic peptidse for tumour treatment.

### The microchip-based protocol reveals the immunopeptidome landscape in patient-derived organoids

The microchip technology was challenged with as few as $6 \times 10^6$ cells from patient-derived organoids (PDOs). We selected two patients from an on-going precision medicine study for urological cancers, a nephrectomy sample containing both benign and cancer tissue from clear cell renal cell carcinoma (ccRCC) patient and 1x1 cm sample from bladder cancer patient, were further processed as 3D primary organoid cultures.

Applying the developed microchip technology and a stringent false discovery rate threshold of 1% for peptide and protein identification, we were able to identify a total of 576 and 2089 unique peptides in ccRCC and bladder PDOs respectively (Fig. 11A). The number of retrieved peptides differed between the two samples, with bladder samples resulting in more peptides than ccRCC samples. It is well known that HLA expression influences the amount of isolated HLA-I peptides [22A], and consistent with this, flow cytometry analysis revealed higher surface levels of HLA-A, HLA-B, and HLA-C in our bladder than in our ccRCC samples, explaining the different yields of retrieved peptides from our samples. Analysis of the peptides showed a preference for 9- to 12mers (56.4% in ccRCC and 47.9% in bladder tumours) with an enrichment in the 9mer population, in line with the typical length distribution of ligandome analysis [33] (Fig. 11B). To discriminate between HLA-I binders and contaminants, Gibbs clustering and NetMHC4.0 analysis was performed. First, deconvolution of the 9mers showed that 55% and 67% in bladder and ccRCC PDOs, respectively, matched at least one of the patients' HLA alleles. Next, NetMHC4.0 was applied to all 9mers identified in our data set. Among them, 46% and 69% were predicted to be binders of the specific HLA of the patients. Next, the source proteins present in both our data sets were investigated. To this end, Gene Ontology (GO) enrichment analysis was performed. Consistent with our previous observations and published data, both samples showed an enrichment in intracellular and nuclear proteins interacting with RNA and involved in catabolic/metabolic processes.

To demonstrate that technology can be exploited for the rapid development of therapeutic cancer vaccines, we set up a killing assay. We focused our analysis on ccRCC samples. We used transcriptomics levels to select putative tumour antigens using PBMC and healthy kidney tissue as reference sets. Next, PBMCs from healthy volunteers were pulsed with the selected peptides, and CD8+ T cells isolated from those cells were used in the assay. T cells pulsed with the peptide EVAQPGPSNR (gene name HSPG2) showed about 10% specific cytolysis.

Finally, we sought to investigate the recall T cells response in the ccRCC patient. To this end, unfractionated PBMCs derived from the patient were in vitro stimulated with the peptide EVAQPGPSNR whereas unstimulated PBMCs were adopted as control. The derived CD8+T cells were then added to the ccRCC PDO showing about a 7% increase in killing activity compared to the control group.

**Peptides derive from patient organoids were shown to be therapeutically effective *in vivo* studies**

The list of peptides was analysed with HEX. First, the software prioritized the peptides that were concurrent strong binders (cut off IC50 range 50nM-500nM according to NetMHC4.0) and that showed higher weighted alignment score (cut off 0.8-1 normalized weighted alignment score). This latter focuses the peptide's similarity in the area of interaction that most likely will engage the TCR of CD8+ T cells, in order to induce mediated immune response; the resultant peptides are then further categorized based on their overall percentage of identity to pathogen-derived antigens/peptides and $IC_{50}$ binding affinity score. The ultimate output consisted in thirteen peptides with their counterpart pathogenic peptides (Table 4).

To determine the peptide immunogenicity, mice were pre-immunized with subcutaneous injection of each single peptide in presence of an adjuvant Poly(I:C); a group of mice was injected either with Poly(I:C) alone or saline as control as well. The splenocytes from those mice were harvested and tested for IFNγ production upon specific stimuli (Table 5) in an ELISpot assay.

We then sought to verify whether the candidate peptides could be exploited as cancer vaccine for treatment of established tumors. To this end, we adopted PeptiCRAd, our previously developed cancer vaccine platform, that combines oncolytic adenoviruses with capsid attached (via a poly-lysine linker) poly-lysine modified peptides. The adenovirus used here is VALO-mD901 i.e., genetically modified to express murine OX40L and CD40L and previously shown to elicit tumor growth control and systemic antitumor response in murine melanoma model. Therefore, Balb/c mice were subcutaneously injected with the syngeneic tumor model CT26 in the left and right flank. (Day 0, Fig12 A). When the tumors were established (Day 7, Fig.12A), Valo-mD901 was coated with a pair of each poly-lysine-modified peptide in our list (PeptiCRAd1, PeptiCRAd2, PeptiCRAd3, Table 6)) and injected intratumorally only in the right tumor. PeptiCRAd4 consisted of Valo-mD901 coated with gp70423-431 (AH1-5). Mock and Valo-mD901 groups were used as control as well. PeptiCRAd 1 and PeptiCRAd2 improved tumor growth control as well as Valo-mD901 in the injected lesions (Fig.12B, right panel). Advantageously, PeptiCRAd1 improved anti-tumor groth control in the untreated tumor in contrast to Valo-mD901 which did not trigger effect.

**Summary**

HEX is a bioinformatic tool that analyzes peptide sequences and compares them to a curated database of pathogen-derived viral proteomes looking for sequences with high similarity.

Since presented peptides are mainly involved in the interaction with the anchor residues of the MHC [25], our software returns an alignment score that is positionally weighted in order to prioritize the similarities occurring in the central section of the peptide, that is the position which is most involved in the interaction with the TCR [26]. Furthermore, in order to increase the chances that our target is a presented epitope, we ranked the resulting peptides according to the binding affinity for the chosen MHCs using, in one example, the IEDB NetMHC prediction tool API [27]; or NetMHC4.0 or NetMHCpan4.1b as stand alone tools.

We used HEX to identify viral-peptides homologous to three extensively characterized tumor associated antigens.

We found that preimmunization with our selected viral peptides pools, simulated an anti-viral immune status prior to the establishment of the tumor and efficiently slowed down the growth of subcutaneously injected melanoma tumor cells in mice indicating that the pre-exposure to viral-derived peptides can affect the tumor growth.

Next, we observed that the tumor-homologous viral-derived peptides have a similar effect on already established tumors when administrated during tumor progression, indicating that a viral infection occurring during the tumor progression could still impact on its growth.

Taken together our results suggest that the molecular mimicry between viral- and tumor-derived antigens is able to exert a control on the tumor growth even without relying on the pre-exposure or pre-acquired immunity.

Novel ICPIs have significantly improved patient survival in several solid tumors, especially in metastatic melanoma, when compared to the other commonly used therapies such as radiation and chemotherapies. However, despite the improved survival and efficient response rates, it is yet known why some patients do not benefit from ICPI-therapies. Our results indicated that patients with high titer of CMV-specific IgG levels had significantly longer progression free survival.

We observed that PBMCs, from patients with high anti-CMV-IgG titer, reacted with melanoma antigens similar to CMV peptides. This data indicates CMV-seropositivity contributes to a melanoma-specific T-cell immunity, and thus provide a clinical advantage for these patients undergoing ICPI therapy.

Here we show that viral infections could have an impact on the tumor growth and clearance. Additionally, we show the cross-reactivity of cytotoxic T-cells against viral- and homologous tumor-derived antigens selected using HEX. Based on our results we conclude that the molecular mimicry phenomenon could be exploited in the future for the development of novel therapies or, when accompanied with immunotherapies, potentially enhance the antitumor immune effect achieved by these therapies.

Moreover, designing effective tumour rejection and protection strategies requires the reliable identification of tumour peptides binding to HLA-I. The direct identification of peptides from the HLA-I complex still represents the best method. Nevertheless, the immunopeptidomics workflow is relatively complex and thus represents a major bottleneck in the antigen discovery process. Currently, the inability to analyse immunopeptidomes from a small amount of biological materials (e.g., tissue needle biopsy), the sample throughput, the cost and the affinity matrix adopted (which is both laborious and expensive to produce) in the conventional platform have been depicted as the main technical challenges to address.

In this work, we addressed several technical issues hindering the ligandome research, with main focus on the limited availability of material to analyse, on the cost of consumables and on prolonged protocols.

By exploiting the well-characterized biotin-streptavidin interaction to immobilize biotinylated pan-HLA antibody on streptavidin-functionalized surfaces, we were able to replace the conventional technology, based on affinity matrices prepared via crosslinking reactions with a microchip platform. The limitations posed by the paucity of the material (e.g., needle biopsy) inspired the work towards the implementation of a microfluidic protocol. In this work, we employed a custom microchip protocol involving a thiol-ene polymer-based micropillar array as the solid support for further biofunctionalization that enabled performing the entire IP procedure on a single microfluidic chip.

The validation of the identified peptides in an *in vitro* killing assay confirmed that the peptides identified when working the invention were actually presented on the JY cell surface, as they were killed in a specific CD8+ T cell-dependent fashion. The peptide ILDKKVEKV (SEQ ID NO: 3) found in our data set elicited the higher percentage of specific cytolysis.

Our approach thus offers an unexplored tool for immune-affinity purification.

**REFERENCES**

20. C. Capasso, M. Hirvinen, M. Garofalo, D. Romaniuk, L. Kuryk, T. Sarvela, A. Vitale, M. Antopolsky, A. Magarkar, T. Viitala, T. Suutari, A. Bunker, M. Yliperttula, A. Urtti, V. Cerullo, Oncolytic adenoviruses coated with MHC-I tumor epitopes increase the antitumor immunity and efficacy against melanoma, Oncoimmunology 5, e1105429 (2016).

21. D. Weinstein, J. Leininger, C. Hamby, B. Safai, Weinstein 2014 Diagnostic and Prognostic biomarkers in melanoma, J. Clin. Aesthet. Dermatol. 7, 13-24 (2014).

25. J. Sidney, E. Assarsson, C. Moore, S. Ngo, C. Pinilla, A. Sette, B. Peters, Quantitative peptide binding motifs for 19 human and mouse MHC class i molecules derived using positional scanning combinatorial peptide libraries, Immunome Res. 4, 2 (2008).

26. A. K. Sharma, J. J. Kuhns, S. Yan, R. H. Friedline, B. Long, R. Tisch, E. J. Collins, Class I Major Histocompatibility Complex Anchor Substitutions Alter the Conformation of T Cell Receptor Contacts, J. Biol. Chem. 276, 21443-21449 (2001).

27. M. Andreatta, M. Nielsen, Gapped sequence alignment using artificial neural networks: Application to the MHC class i system, Bioinformatics 32, 511-517 (2016).

28. P. C. Rosato, S. Wijeyesinghe, J. M. Stolley, C. E. Nelson, R. L. Davis, L. S. Manlove, C. A. Pennell, B. R. Blazar, C. C. Chen, M. A. Geller, V. Vezys, D. Masopust, Virus-specific memory T cells populate tumors and can be repurposed for tumor immunotherapy, Nat. Commun. 10, 567 (2019).

33. S. Giguère, A. Drouin, A. Lacoste, M. Marchand, J. Corbeil, F. Laviolette, MHC-NP: Predicting peptides naturally processed by the MHC, J. Immunol. Methods 400-401, 30-36 (2013).

3A. Bassani-Sternberg M, Pletscher-Frankild S, Jensen LJ, Mann M. Mass spectrometry of human leukocyte antigen class I peptidomes reveals strong effects of protein abundance and turnover on antigen presentation. Mol Cell Proteomics. 2015;14(3):658-73.

15A. Lee SH, Hu W, Matulay JT, Silva MV, Owczarek TB, Kim K, et al. Tumor Evolution and Drug Response in Patient-Derived Organoid Models of Bladder Cancer. Cell. 2018;173(2):515-28 e17.

22A. Purcell AW, Ramarathinam SH, Ternette N. Mass spectrometry-based identification of MHC-bound peptides for immunopeptidomics. Nat Protoc. 2019;14(6):1687-707.

24A. Jurtz V, Paul S, Andreatta M, Marcatili P, Peters B, Nielsen M. NetMHCpan-4.0: Improved Peptide-MHC Class I Interaction Predictions Integrating Eluted Ligand and Peptide Binding Affinity Data. J Immunol. 2017;199(9):3360-8.

25A. Nielsen M, Andreatta M. NetMHCpan-3.0; improved prediction of binding to MHC class I molecules integrating information from multiple receptor and peptide length datasets. Genome Med. 2016;8(1):33.

26A. Hoof I, Peters B, Sidney J, Pedersen LE, Sette A, Lund O, et al. NetMHCpan, a method for MHC class I binding prediction beyond humans. Immunogenetics. 2009;61(1):1-13.

Table 1

| Name of the protein | Sequence | |
|---|---|---|
| **TYR (208-216)** | **LPWHRLFLL** | SEQ ID NO: 35 |
| Viral peptides - POOL 1: | | |
| >GI\|9626011\|REF\|NP_040258,1\| UNNAMED PROTEIN PRODUCT [SAIMIRIINE HERPESVIRUS 2] | FAWPRLFEL | SEQ ID NO: 36 |
| >GI\|23309025\|REF\|NP_694680,1\|GUANYLYLTRANSFERASE [MAMMALIAN ORTHOREOVIRUS 3] | LRWTRLALL | SEQ ID NO: 37 |
| >SP\|P16752\|UL79_HCMVA PROTEIN UL79 OS=HUMAN CYTOMEGALOVIRUS (STRAIN AD169) GN=UL79 PE=3 SV=1 | LYGHRLFRL | SEQ ID NO: 38 |
| >SP\|Q49P94\|GAAP_VACCL GOLGI ANTI-APOPTOTIC PROTEIN OS=VACCINIA VIRUS (STRAIN LISTER) GN=L6 PE=1 SV=1 | LFLHLLQLL | SEQ ID NO: 39 |
| **TRP2 (180-188)** | **SVYDFFVWL** | SEQ ID NO: 40 |

(continued)

Viral peptides - POOL 2:

| | | |
|---|---|---|
| >GI\|46852141\|REF\|YP_012613,1\| RNA DEPENDENT RNA POLYMERASE [HUMAN METAPNEUMOVIRUS] | LNYDFFEAL | SEQ ID NO: 41 |
| >SP\|Q38SQ8\|HEMA_183A8 HEMAGGLUTININ OS=INFLUENZA A VIRUS (STRAIN A/HONG KONG/5/1983 H3N2) GN=HA PE=3 SV=1 | SVNSFFSRL | SEQ ID NO: 42 |
| >GI\|66275873\|REF\|YP_232958,1\| VIRAL CORE CYSTEINE PROTEINASE [VACCINIA VIRUS] | KVYTFFKFL | SEQ ID NO: 43 |
| >GI\|9625900\|REF\|NP_040149,1\| DNA PACKAGING PROTEIN UL32 [HUMAN HERPESVIRUS 3] | SNYSFFVQA | SEQ ID NO: 44 |
| **hGP100 (25-33)** | **KVPRNQDWL** | SEQ ID NO: 45 |

Vial peptides - POOL 3:

| | | |
|---|---|---|
| >GI\|389656439\|REF\|YP_006393315,1\|MAJOR CAPSID PROTEIN [HUMAN PAPILLOMAVIRUS TYPE 144] | KVPLNADVL | SEQ ID NO: 46 |
| >GI\|253756606\|REF\|YP_003038519,1\| ORF1A POLYPROTEIN [HUMAN ENTERIC CORONAVIRUS STRAIN 4408] | KATRNNCWL | SEQ ID NO: 47 |
| >SP\|Q3KSU8\|DEN_EBVG DENEDDYLASE OS=EPSTEIN-BARR VIRUS (STRAIN GD1) GN=BPLF1 PE=3 SV=1 | AVARNTDIL | SEQ ID NO: 48 |
| >SP\|Q66849\|POLG_EC09H GENOME POLYPROTEIN OS=ECHOVIRUS 9 (STRAIN HILL) PE=3 SV=3 | IVVRNHDDL | SEQ ID NO: 49 |

Table 2

| ID | Tumor Antigen | Sequence | ID | Viral Protein | Sequence |
|---|---|---|---|---|---|
| T1 | hTERT (934-942) | LLDTRTLEV (SEQ ID NO: 50) | V1 | hCMV Envelope Glycoprotein H, gH | MLDRRTVEM (SEQ 10 NO:55) |
| T2 | MAGE A10 (353-361) | AMASASSSA (SEQ 10 NO: 51 | V2 | hCMV 65kDa phosphoprotein, pp65 | AMAGASTSA (SEQ ID NO:56) |
| T3 | FINC (2009-2017) | EILDVPSTV (SEQ ID NO: 52) | V3 | hCMV Uncharacterized protein UL42 | DMMEMPATM (SEQ: ID: NO: 57) |
| T4 | CSPG4 (22-30) | MLARLASAA (SEQ ID NO: 53 | V4 | hCMV Major Capsid Protein, MCP | FLTRLAEAA (SEQ ID NO: 58) |
| T5 | MAGE A10 (251-259) | NMMGLYDGM (SEQ 1D NO:54) | V5 | hCMV 55kOa immediate-early protein, IE1 | CMMTMYGGI (SEQ 10 NO: 59) |

Table 3

|  | Total antibody consumed | antibody moles | #of antibody molecules | Total amount of cells to use |
|---|---|---|---|---|
| Standard procedure | 10mg | 64.5 nmol | $3.88 \times 10^{16}$ | $1 \times 10^9$ |
| Microchip | 45$\mu$g | 0.29 nmol | $1.74 \times 10^{14}$ | $4.5 \times 10^6$ |

Table 4

| Uniprot ID | Peptide sequence | Pathogen species | Viral Peptide |
|---|---|---|---|
| O88738-3 | SYHPALNAI (SEQ ID NO: 1) | Molluscum contagiosum virus subtype 1 | SYHAALNAL |
| Q9QXZ0 | AFHSSRTSL (SEQ ID NO: 2) | Human adenovirus A serotype 31 | HFSTSRTSL |
| Q3TWW8 | SYSDMKRAL (SEQ ID NO: 3) | Cercopithecine herpesvirus 1 | AYQDTKRAL |
| P70452 | NYNSVNTRM (SEQ ID NO: 4) | Human herpesvirus 7 | FYNSVNTRN |
| Q80TP3 | SYLTSASSL (SEQ ID NO: 5) | Influenza A virus | TIWTSASSI |
| Q8VCF0 | SYLPPGTSL (SEQ ID NO: 6) | Epstein-Barr virus | TYLPPSTSS |
| O70405 | FYEKNKTLV (SEQ ID NO: 7) | Orf virus | NYYKNKSLV |
| Q9D1R1 | FYKNGRLAV (SEQ ID NO: 8) | Human adenovirus F serotype 41 | AYMNGRVAV |
| Q91XE7 | KGPNRGVII (SEQ ID NO: 9) | Variola virus | KNPNRFVIF |
| Q6URW6-2 | LYKESLSRL (SEQ ID NO: 10) | Human cytomegalovirus | LYLETLSRI |
| Q9JL70 | RYLPAPTAL (SEQ ID NO: 11) | Influenza C virus | RNMPAATAL |
| O54692 | KYIPAARHL (SEQ ID NO: 12) | Human cytomegalovirus | SHQPAARRL |
| P54775 | YYVRILSTI (SEQ ID NO: 13) | Molluscum contagiosum virus subtype 1 | YVFRLLSTI |
| P54775 | SYRDVIQEL (SEQ ID NO: 14) | Human cytomegalovirus | RYADVIQEV |
| Q61036 | KFYDSKETV (SEQ ID NO: 15) | Human adenovirus A serotype 18 | NFYNSKETV |

Table 5.

| Group 1 | Group 4 | Group 7 |
|---|---|---|
| 1. SYHPALNAI (SEQ ID NO:1) | 10. FYEKNKTLV (SEQ ID NO: 7) | 18. SGPNRFILI (SEQ ID NO: 18) |
| 2. SYLTSASSL (SEQ ID NO: 5) | 11. KGPNRGVII (SEQ ID NO: 9) | 19. SYIIGTSSV (SEQ ID NO: 19) |
| 3. YYVRILSTI (SEQ ID NO: 13) | 12. FYKNGRLAV (SEQ ID NO: 8) | 20. RGPVYREF (SEQ ID NO: 20) |
| **Group 2** | **Group 5** | **Group 8** |
| 4. SYLPPGTSL (SEQ ID NO: 6) | 13. LYKESLSRL (SEQ ID NO: 10) | 21. FYATIIHDL (SEQ ID NO: 21) |
| 5. RYLPAPTAL (SEQ ID NO: 11) | 14. SYROVIQEL (SEQ ID NO: 14) | 22. GYMTPGLTV (SEQ ID NO: 22) |
| 6. KYIPAARHL (SEQ ID NO: 12) | 15. KFYDSKETV (SEQ ID NO: 15) | 23. SYLIGRQKI (SEQ ID NO: 23) |
| **Group 3** | **Group 6** | **Group 9** |
| 7. AFHSSRTSL (SEQ ID NO: 2) | 16. KYLNVREAV (SEQ ID NO: 16) | 24. AGASRIIGI (SEQ ID NO: 24) |
| 8. NYNSVNTRM (SEQ ID NO: 4) | 17. HYLPDLHHM (SEQ ID NO: 17) | 25. QPEYIERL (SEQ ID NO: 25) |
| 9. SYSDMKRAL (SEQ ID NO: 3) | | 26. SYIHQRYIL (SEQ ID NO: 26) |

**EP 4 147 047 B1**

Table 6.

| Name of Peptide | Peptide Sequence | Net charge pH7 | Poly-lysine peptide | Net charge pH7 |
|---|---|---|---|---|
| **Peptide 1** | SYLPPGTSL (SEQ ID NO: 6) | 0 | KKKKKKSYLPPGTSL (SEQ ID NO: 28) | 6 |
| **Peptide 2** | RYLPAPTAL (SEQ ID NO: 11) | 1 | KKKKKKRYLPAPTAL (SEQ ID NO: 29) | 7 |
| **Peptide 3** | KYIPAARHL (SEQ ID NO: 12) | 2.1 | KKKKKKYIPAARHL (SEQ ID NO: 30) | 7.1 |
| **Peptide 4** | LYKESLSRL (SEQ ID NO: 10) | 1 | KKKKKKLYKESLSRL (SEQ ID NO: 31) | 7 |
| **Peptide 5** | KYLNVREAV (SEQ ID NO: 16) | 1 | KKKKKKKYLNVREAV (SEQ ID NO: 32) | 6 |
| **Peptide 6** | FYATIIHDL (SEQ ID NO: 21) | -0.9 | KKKKKKKFYATIIHDL (SEQ ID NO: 33) | 6.1 |
| **Peptide 7** | SPSYAYHQF (SEQ ID NO: 27) | 0.1 | KKKKKKSPSYAYHQF (SEQ ID NO: 34) | 6.1 |

**Claims**

1. A method for tumour-specific antigen identification comprising:

   i) dissolving or suspending a sample of tumour in a fluid;
   ii) passing said fluid through a microfluidic device for tumour-specific antigen identification comprising: at least one flow-through channel containing a plurality of micropillars arranged in an array to which there is attached at least one molecule, or at least one complex, that has bound thereto at least one anti-Major Histocompatibility Complex antibody or at least one anti-pan-Human Leukocyte Antigen antibody whereby a peptide:Major Histocompatibility Complex (pMHC) in a sample flowing through said channel can be extracted from said sample using said at least one antibody.
   iii) binding to said at least one antibody at least one pMHC in said sample;
   iv) removing said at least one bound pMHC of part iii) from said device;
   v) comparing said peptide of said bound pMHC with a library of pathogen-derived antigens to determine if said peptide shows homology, by way of a comparison of sequence structures, with at least one pathogenic antigen, or part thereof, and where greater than 60% homology exists;
   vi) identifying said peptide as a tumour-specific antigen for use in cancer therapy.

2. The method according to claim 1 wherein said library of pathogen-derived antigens comprises a curated library of known pathogenic antigens.

3. The method according to claims 1 or 2 wherein said pathogenic antigens are human pathogenic antigens.

4. The method according to any one of claims 1 - 3 wherein said pathogenic antigens are viruses.

5. The method according to any one of claims 1 - 4 wherein said pathogenic antigens are derived from at least one virus, including any combination thereof, selected from the group comprising:
   Abyssoviridae; Ackermannviridae; Actantavirinae; Adenoviridae; Agantavirinae; Aglimvirinae; Alloherpesviridae; Alphaflexiviridae; Alphaherpesvirinae; Alphairidovirinae; Alphasatellitidae; Alphatetraviridae; Alvernaviridae; Amalgaviridae; Amnoonviridae; Ampullaviridae; Anelloviridae; Arenaviridae; Arquatrovirinae; Arteriviridae; Artoviridae; Ascoviridae; Asfarviridae; Aspiviridae; Astroviridae; Autographivirinae; Avsunviroidae; Avulavirinae; Bacilladnaviridae; Baculoviridae; Barnaviridae; Bastillevirinae; Bclasvirinae; Belpaoviridae; Benyviridae; Betaflexiviridae; Betaherpesvirinae; Betairidovirinae; Bicaudaviridae; Bidnaviridae; Birnaviridae; Bornaviridae; Botourmiaviridae; Brockvirinae; Bromoviridae; Bullavirinae; Caliciviridae; Calvusvirinae; Carmotetraviridae; Caulimoviridae; Ceronivirinae; Chebruvirinae; Chordopoxvirinae; Chrysoviridae; Chuviridae; Circoviridae; Clavaviridae; Closteroviridae; Comovirinae; Coronaviridae; Corticoviridae; Crocarterivirinae; Cruliviridae; Crustonivirinae; Cvivirinae; Cystoviridae; Dclasvirinae; Deltaflexiviridae; Densovirinae; Dicistroviridae; Endornaviridae; Entomopoxvirinae; Equarterivirinae; Eucampyvirinae; Euroniviridae; Filoviridae; Fimoviridae; Firstpapillomavirinae; Flaviviridae; Fuselloviridae; Gammaflexiviridae; Gammaherpesvirinae; Geminialphasatellitinae; Geminiviridae; Genomoviridae; Globuloviridae; Gokushovirinae; Guernseyvirinae; Guttaviridae; Hantaviridae; Hepadnaviridae; Hepeviridae; Herelleviridae; Heroarterivirinae; Herpesviridae; Hexponivirinae; Hypoviridae; Hytrosaviridae; Iflaviridae; Inoviridae; Iridoviridae; Jasinkavir-

23

inae; Kitaviridae; Lavidaviridae; Leishbuviridae; Letovirinae; Leviviridae; Lipothrixviridae; Lispiviridae; Luteoviridae; Malacoherpesviridae;; Mammantavirinae; Marnaviridae; Marseilleviridae; Matonaviridae; Mccleskeyvirinae; Mclasvirinae; Medioniviridae; Medionivirinae; Megabirnaviridae; Mesoniviridae; Metaparamyxovirinae; Metaviridae; Microviridae; Mimiviridae; Mononiviridae; Mononivirinae; Mymonaviridae; Myoviridae; Mypoviridae; Nairoviridae; Nanoalphasatellitinae; Nanoviridae; Narnaviridae; Nclasvirinae; Nimaviridae; Nodaviridae; Nudiviridae; Nyamiviridae; Nymbaxtervirinae; Okanivirinae; Orthocoronavirinae; Orthomyxoviridae; Orthoparamyxovirinae; Orthoretrovirinae; Ounavirinae; Ovaliviridae; Papillomaviridae; Paramyxoviridae; Partitiviridae; Parvoviridae; Parvovirinae; Pclasvirinae; Peduovirinae; Peribunyaviridae; Permutotetraviridae; Phasmaviridae; Phenuiviridae; Phycodnaviridae; Picobirnaviridae; Picornaviridae; Picovirinae; Piscanivirinae; Plasmaviridae; Pleolipoviridae; Pneumoviridae; Podoviridae; Polycipiviridae; Polydnaviridae; Polyomaviridae; Portogloboviridae; Pospiviroidae; Potyviridae; Poxviridae; Procedovirinae; Pseudoviridae; Qinviridae; Quadriviridae; Quinvirinae; Regressovirinae; Remotovirinae; Reoviridae; Repantavirinae; Retroviridae; Rhabdoviridae; Roniviridae; Rubulavirinae; Rudiviridae; Sarthroviridae; Secondpapillomavirinae; Secoviridae; Sedoreovirinae; Sepvirinae; Serpentovirinae; Simarterivirinae; Siphoviridae; Smacoviridae; Solemoviridae; Solinviviridae; Sphaerolipoviridae; Spinareovirinae; Spiraviridae; Spounavirinae; Spumaretrovirinae; Sunviridae; Tectiviridae; Tevenvirinae; Tiamatvirinae; Tobaniviridae; Togaviridae; Tolecusatellitidae; Tombusviridae; Torovirinae; Tospoviridae; Totiviridae;; Tristromaviridae; Trivirinae; Tunavirinae; Tunicanivirinae; Turriviridae; Twortvirinae; Tymoviridae; Variarterivirinae; Vequintavirinae; Virgaviridae; Wupedeviridae; Xinmoviridae; Yueviridae; and Zealarterivirinae.

6. The method according to any one of claims 1 - 5 wherein said pathogenic antigen is a cytomegalovirus antigen or Epstein-Barr virus antigen or, a Herpesvirus antigen, or a Poxvirus antigen, or a Hepadnavirus antigen, or an Influenza virus antigen, or a Coronavirus antigen, or a Hepatitis virus antigen, or a HIV antigen, or Bunyavirus antigen.

7. The method according to any one of claims 1 - 6 wherein comparing said peptide of said bound pMHC with a library of pathogenic antigens involves peptide scoring and/or alignment scoring to determine said homology.

8. The method according to any one of claims 1-7 wherein comparing said peptide of said bound pMHC with a library of pathogenic antigens involves determining:

    a) the similarity or identity of the entire sequence structures of said peptide and said pathogenic antigens; and/or
    b) the similarity or identity of key amino acids in key binding sites of said peptide and said pathogenic antigens; and/or
    c) the most number of similar or identical key amino acids in key binding sites of said peptide and said pathogenic antigens.

9. A device for tumour-specific antigen identification comprising:

    a microfluidic device comprising at least one flow-through channel containing a plurality of micropillars arranged in an array to which there is attached at least one molecule, or at least one complex, that has bound thereto at least one anti-Major Histocompatibility Complex antibody or at least one anti-pan-Human Leukocyte Antigen (HLA) antibody whereby a peptide:Major Histocompatibility Complex (pMHC) in a sample flowing through said channel can be extracted from said sample using said at least one antibody ; and
    a processor adapted for identifying said peptide bound to said Major Histocompatibility Complex and comparing said peptide of said bound pMHC with a library of pathogen-derived peptide antigens to determine if said peptide bound or that was bound to said Major Histocompatibility Complex shows homology, by way of a comparison of sequence structures, with at least one pathogenic antigen, or part thereof, and where greater than 60% homology exists; indicating said peptide bound or that was bound to said Major Histocompatibility Complex as a tumour-specific antigen for use in cancer therapy.

10. The device according to claim 9 wherein said anti-pan-HLA antibody is selected from the group comprising: MHC class I A, B, and C.

11. The device according to claim 9 wherein said anti-pan-HLA antibody is selected from the group comprising: MHC class II DP, DM, DO, DQ, and DR.

12. The device according to any one of claims 9 - 11 wherein said antibody is anti-human.

13. The device according to any one of claims 9-12 wherein said molecule or complex is a complex of thiol and alkene functional groups.

**14.** The device according to any one of claims 9-13 wherein said molecule or complex comprises biotin and streptavidin.

**15.** The device according to any one of claims 9-14 wherein said molecule or complex comprises more than one antibody bound to streptavidin.

**16.** A method of stratifying patients for checkpoint inhibitor cancer treatment comprising:

i) dissolving or suspending a sample of tumour from a patient in a fluid;
ii) passing said fluid through a microfluidic device for tumour-specific antigen identification comprising: at least one flow-through channel containing a plurality of micropillars arranged in an array to which there is attached at least one molecule, or at least one complex, that has bound thereto at least one anti-Major Histocompatibility Complex antibody or at least one anti-pan-Human Leukocyte Antigen antibody whereby a peptide:Major Histocompatibility Complex (pMHC) in a sample flowing through said channel can be extracted from said sample using said at least one antibody;
iii) binding to said at least one antibody at least one pMHC in said sample;
iv) removing said at least one bound pMHC of part iii) from said device;
v) comparing said peptide of said bound pMHC with a library of human pathogen-antigens to determine if said peptide shows homology, by way of a comparison of sequence structures, with at least one human pathogenic antigen, or part thereof, and where greater than 60% homology exists;
vi) identifying said peptide as a tumour-specific antigen; and
vii) when said peptide is found, stratifying said patient as suitable for treatment using an effective amount of at least one checkpoint inhibitor.

**17.** The method according to claim 16 wherein said cancer is selected from the group comprising: nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/-lymphoma, neuroma, von Hippel-Lindau disease, Zollinger-Ellison syndrome, adrenal cancer, anal cancer, bile duct cancer, bladder cancer, ureter cancer, oligodendroglioma, neuroblastoma, meningioma, spinal cord tumor, osteochondroma, chondrosarcoma, Ewing's sarcoma, cancer of unknown primary site, carcinoid, carcinoid of gastro-intestinal tract, fibrosarcoma, breast cancer, Paget's disease, cervical cancer, esophagus cancer, gall bladder cancer, head cancer, eye cancer, neck cancer, kidney cancer, Wilms' tumor, liver cancer, Kaposi's sarcoma, prostate cancer, testicular cancer, Hodgkin's disease, non-Hodgkin's lymphoma, skin cancer, mesothelioma, multiple myeloma, ovarian cancer, endocrine pancreatic cancer, glucagonoma, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer and tonsil cancer.

**Patentansprüche**

**1.** Verfahren zur Identifizierung tumorspezifischer Antigene, umfassend:

i) Lösen oder Suspendieren einer Tumorprobe in einem Fluid;
ii) Leiten des Fluids durch eine mikrofluidische Vorrichtung zur Identifizierung tumorspezifischer Antigene, umfassend: zumindest einen Durchflusskanal, der eine Vielzahl von Mikropfeilern enthält, die in einem Array angeordnet sind, an das zumindest ein Molekül oder zumindest ein Komplex angehängt ist, das/der zumindest einen daran gebundenen Anti-Haupthistokompatibilitätskomplex-Antikörper oder zumindest einen daran gebundenen Anti-Pan-Human-Leukozyten-Antigen-Antikörper aufweist, wodurch ein Peptid:Haupthistokompatibilitätskomplex (pMHC) in einer Probe, die durch den Kanal fließt, aus der Probe unter Verwendung des zumindest einen Antikörpers extrahiert werden kann,
iii) Binden von zumindest einem pMHC in der Probe an den zumindest einen Antikörper;
iv) Entfernen des zumindest einen gebundenen pMHC von Teil iii) aus der Vorrichtung;
v) Vergleichen des Peptids des gebundenen pMHC mit einer Bibliothek von Pathogen-abgeleiteten Antigenen, um zu bestimmen, ob das Peptid Homologie zeigt, durch einen Vergleich von Sequenzstrukturen, mit zumindest einem pathogenen Antigen, oder Teil davon, und wo mehr als 60 % Homologie existiert;

vi) Identifizieren des Peptids als ein tumorspezifisches Antigen zur Verwendung bei Krebstherapie.

2. Verfahren nach Anspruch 1, wobei die Bibliothek von Pathogen-abgeleiteten Antigenen eine kuratierte Bibliothek von bekannten pathogenen Antigenen umfasst.

3. Verfahren nach Anspruch 1 oder 2, wobei die pathogenen Antigene humane pathogene Antigene sind.

4. Verfahren nach einem der Ansprüche 1 - 3, wobei die pathogenen Antigene Viren sind.

5. Verfahren nach einem der Ansprüche 1 - 4, wobei die pathogenen Antigene abgeleitet sind von zumindest einem Virus, einschließlich einer beliebigen Kombination davon, ausgewählt aus der Gruppe umfassend:
Abyssoviridae; Ackermannviridae; Actantavirinae; Adenoviridae; Agantavirinae; Aglimvirinae; Alloherpesviridae; Alphaflexiviridae; Alphaherpesvirinae; Alphairidovirinae; Alphasatellitidae; Alphatetraviridae; Alvernaviridae; Amalgaviridae; Amnoonviridae; Ampullaviridae; Anelloviridae; Arenaviridae; Arquatrovirinae; Arteriviridae; Artoviridae; Ascoviridae; Asfarviridae; Aspiviridae; Astroviridae; Autographivirinae; Avsunviroidae; Avulavirinae; Bacilladnaviridae; Baculoviridae; Barnaviridae; Bastillevirinae; Bclasvirinae; Belpaoviridae; Benyviridae; Betaflexiviridae; Betaherpesvirinae; Betairidovirinae; Bicaudaviridae; Bidnaviridae; Birnaviridae; Bornaviridae; Botourmiaviridae; Brockvirinae; Bromoviridae; Bullavirinae; Caliciviridae; Calvusvirinae; Carmotetraviridae; Caulimoviridae; Ceronivirinae; Chebruvirinae; Chordopoxvirinae; Chrysoviridae; Chuviridae; Circoviridae; Clavaviridae; Closteroviridae; Comovirinae; Coronaviridae; Corticoviridae; Crocarterivirinae; Cruliviridae; Crustonivirinae; Cvivirinae; Cystoviridae; Dclasvirinae; Deltaflexiviridae; Densovirinae; Dicistroviridae; Endornaviridae; Entomopoxvirinae; Equarterivirinae; Eucampyvirinae; Euroniviridae; Filoviridae; Fimoviridae; Firstpapillomavirinae; Flaviviridae; Fuselloviridae; Gammaflexiviridae; Gammaherpesvirinae; Geminialphasatellitinae; Geminiviridae; Genomoviridae; Globuloviridae; Gokushovirinae; Guernseyvirinae; Guttaviridae; Hantaviridae; Hepadnaviridae; Hepeviridae; Herelleviridae; Heroarterivirinae; Herpesviridae; Hexponivirinae; Hypoviridae; Hytrosaviridae; Iflaviridae; Inoviridae; Iridoviridae; Jasinkavirinae; Kitaviridae; Lavidaviridae; Leishbuviridae; Letovirinae; Leviviridae; Lipothrixviridae; Lispiviridae; Luteoviridae; Malacoherpesviridae; Mammantavirinae; Marnaviridae; Marseilleviridae; Matonaviridae; Mccleskeyvirinae; Mclasvirinae; Medioniviridae; Medionivirinae; Megabirnaviridae; Mesoniviridae; Metaparamyxovirinae; Metaviridae; Microviridae; Mimiviridae; Mononiviridae; Mononivirinae; Mymonaviridae; Myoviridae; Mypoviridae; Nairoviridae; Nanoalphasatellitinae; Nanoviridae; Narnaviridae; Nclasvirinae; Nimaviridae; Nodaviridae; Nudiviridae; Nyamiviridae; Nymbaxtervirinae; Okanivirinae; Orthocoronavirinae; Orthomyxoviridae; Orthoparamyxovirinae; Orthoretrovirinae; Ounavirinae; Ovaliviridae; Papillomaviridae; Paramyxoviridae; Partitiviridae; Parvoviridae; Parvovirinae; Pclasvirinae; Peduovirinae; Peribunyaviridae; Permutotetraviridae; Phasmaviridae; Phenuiviridae; Phycodnaviridae; Picobirnaviridae; Picornaviridae; Picovirinae; Piscanivirinae; Plasmaviridae; Pleolipoviridae; Pneumoviridae; Podoviridae; Polycipiviridae; Polydnaviridae; Polyomaviridae; Portogloboviridae; Pospiviroidae; Potyviridae; Poxviridae; Procedovirinae; Pseudoviridae; Qinviridae; Quadriviridae; Quinvirinae; Regressovirinae; Remotovirinae; Reoviridae; Repantavirinae; Retroviridae; Rhabdoviridae; Roniviridae; Rubulavihnae; Rudiviridae; Sarthroviridae; Secondpapillomavirinae; Secoviridae; Sedoreovirinae; Sepvirinae; Serpentovirinae; Simarterivirinae; Siphoviridae; Smacoviridae; Solemoviridae; Solinviviridae; Sphaerolipoviridae; Spinareovirinae; Spiraviridae; Spounavirinae; Spumaretrovirinae; Sunviridae; Tectiviridae; Tevenvirinae; Tiamatvirinae; Tobaniviridae; Togaviridae; Tolecusatellitidae; Tombusviridae; Torovirinae; Tospoviridae; Totiviridae; Tristromaviridae; Trivirinae; Tunavirinae; Tunicanivirinae; Turriviridae; Twortvirinae; Tymoviridae; Variarterivirinae; Vequintavirinae; Virgaviridae; Wupedeviridae; Xinmoviridae; Yueviridae; und Zealarterivirinae.

6. Verfahren nach einem der Ansprüche 1 - 5, wobei das pathogene Antigen ein Cytomegalovirus-Antigen oder Epstein-Barr-Virus-Antigen oder ein Herpesvirus-Antigen oder ein Poxvirus-Antigen oder ein Hepadnavirus-Antigen oder ein Influenzavirus-Antigen oder ein Coronavirus-Antigen oder ein Hepatitisvirus-Antigen oder ein HIV-Antigen oder Bunyavirus-Antigen ist.

7. Verfahren nach einem der Ansprüche 1 - 6, wobei das Vergleichen des Peptids des gebundenen pMHC mit einer Bibliothek von pathogenen Antigenen Peptid-Scoring und/oder Alignment-Scoring involviert, um die Homologie zu bestimmen.

8. Verfahren nach einem der Ansprüche 1-7, wobei das Vergleichen des Peptids des gebundenen pMHC mit einer Bibliothek von pathogenen Antigenen Bestimmen von Folgendem involviert:

a) der Ähnlichkeit oder Identität der gesamten Sequenzstrukturen des Peptids und der pathogenen Antigene; und/oder

b) der Ähnlichkeit oder Identität von Schlüsselaminosäuren in Schlüsselbindungsstellen des Peptids und der pathogenen Antigene; und/oder

c) der höchsten Anzahl an ähnlichen oder identischen Schlüsselaminosäuren in Schlüsselbindungsstellen des Peptids und der pathogenen Antigene.

9. Vorrichtung zur Identifizierung tumorspezifischer Antigene, umfassend:

eine mikrofluidische Vorrichtung, die zumindest einen Durchflusskanal umfasst, der eine Vielzahl von Mikropfeilern enthält, die in einem Array angeordnet sind, an das zumindest ein Molekül oder zumindest ein Komplex angehängt ist, das/der zumindest einen daran gebundenen Anti-Haupthistokompatibilitätskomplex-Antikörper oder zumindest einen daran gebundenen Anti-Pan-Human-Leukozyten-Antigen(HLA-)Antikörper aufweist, wodurch ein Peptid:Haupthistokompatibilitätskomplex (pMHC) in einer Probe, die durch den Kanal fließt, aus der Probe unter Verwendung des zumindest einen Antikörpers extrahiert werden kann; und

einen Prozessor, der ausgelegt ist, um das an den Haupthistokompatibilitätskomplex gebundene Peptid zu identifizieren und das Peptid des gebundenen pMHC mit einer Bibliothek von Pathogen-abgeleiteten Peptidantigenen zu vergleichen, um zu bestimmen, ob das Peptid, das an den Haupthistokompatibilitätskomplex gebunden ist oder das daran gebunden war, Homologie zeigt, durch einen Vergleich von Sequenzstrukturen mit zumindest einem pathogenen Antigen, oder Teil davon, und wo mehr als 60 % Homologie existiert; wobei das Peptid, das an den Haupthistokompatibilitätskomplex gebunden ist oder das daran gebunden war, als ein tumorspezifisches Antigen zur Verwendung bei Krebstherapie angegeben wird.

10. Vorrichtung nach Anspruch 9, wobei der Anti-Pan-HLA-Antikörper ausgewählt ist aus der Gruppe umfassend: MHC Klasse I A, B und C.

11. Vorrichtung nach Anspruch 9, wobei der Anti-Pan-HLA-Antikörper ausgewählt ist aus der Gruppe umfassend: MHC Klasse II DP, DM, DO, DQ und DR.

12. Vorrichtung nach einem der Ansprüche 9 - 11, wobei der Antikörper anti-human ist.

13. Vorrichtung nach einem der Ansprüche 9-12, wobei das Molekül oder der Komplex ein Komplex aus Thiol und funktionalen Alkengruppen ist.

14. Vorrichtung nach einem der Ansprüche 9-13, wobei das Molekül oder der Komplex Biotin und Streptavidin umfasst.

15. Vorrichtung nach einem der Ansprüche 9-14, wobei das Molekül oder der Komplex mehr als einen an Streptavidin gebundenen Antikörper umfasst.

16. Verfahren zum Stratifizieren von Patienten zur Checkpointinhibitorkrebsbehandlung, umfassend:

i) Lösen oder Suspendieren einer Tumorprobe von einem Patienten in einem Fluid;

ii) Leiten des Fluids durch eine mikrofluidische Vorrichtung zur Identifizierung tumorspezifischer Antigene, umfassend: zumindest einen Durchflusskanal, der eine Vielzahl von Mikropfeilern enthält, die in einem Array angeordnet sind, an das zumindest ein Molekül oder zumindest ein Komplex angehängt ist, das/der zumindest einen daran gebundenen Anti-Haupthistokompatibilitätskomplex-Antikörper oder zumindest einen daran gebundenen Anti-Pan-Human-Leukozyten-Antigen-Antikörper aufweist, wodurch ein Peptid:Haupthistokompatibilitätskomplex (pMHC) in einer Probe, die durch den Kanal fließt, aus der Probe unter Verwendung des zumindest einen Antikörpers extrahiert werden kann;

iii) Binden von zumindest einem pMHC in der Probe an den zumindest einen Antikörper;

iv) Entfernen des zumindest einen gebundenen pMHC von Teil iii) aus der Vorrichtung;

v) Vergleichen des Peptids des gebundenen pMHC mit einer Bibliothek von humanen Pathogen-Antigenen, um zu bestimmen, ob das Peptid Homologie zeigt, durch einen Vergleich von Sequenzstrukturen, mit zumindest einem humanen pathogenen Antigen, oder Teil davon, und wo mehr als 60 % Homologie existiert;

vi) Identifizieren des Peptids als ein tumorspezifisches Antigen; und

vii) wenn das Peptid gefunden wird, Stratifizieren des Patienten als geeignet für Behandlung unter Verwendung einer wirksamen Menge von zumindest einem Checkpointinhibitor.

17. Verfahren nach Anspruch 16, wobei der Krebs ausgewählt ist aus der Gruppe umfassend: Nasopharynxkrebs, Synovialkrebs, hepatozellulären Krebs, Nierenkrebs, Bindegewebskrebs, Melanom, Lungenkrebs, Darmkrebs, Dickdarmkrebs, Rektalkrebs, Kolorektalkrebs, Gehirnkrebs, Halskrebs, Mundhöhlenkrebs, Leberkrebs, Knochenkrebs, Bauchspeicheldrüsenkrebs, Chorionkarzinom, Gastrinom, Phäochromozytom, Prolaktinom, T-Zell-Leukämie/-Lymphom, Neurom, Von-Hippel-Lindau-Erkrankung, Zollinger-Ellison-Syndrom, Nebennierenkrebs, Analkrebs, Gallengangkrebs, Blasenkrebs, Harnleiterkrebs, Oligodendrogliom, Neuroblastom, Meningiom, Rückenmarkstumor, Osteochondrom, Chondrosarkom, Ewing-Sarkom, Krebs unbekannter Primärstelle, Karzinoid, Karzinoid des Gastrointestinaltrakts, Fibrosarkom, Brustkrebs, Paget-Erkrankung, Gebärmutterhalskrebs, Speiseröhrenkrebs, Gallenblasenkrebs, Kopfkrebs, Augenkrebs, Halskrebs, Nierenkrebs, Wilms-Tumor, Leberkrebs, Kaposi-Sarkom, Prostatakrebs, Hodenkrebs, Hodgkin-Erkrankung, Non-Hodgkin-Lymphom, Hautkrebs, Mesotheliom, multiples Myelom, Eierstockkrebs, endokrinen Bauchspeicheldrüsenkrebs, Glukagonom, Nebenschilddrüsenkrebs, Peniskrebs, Hypophysenkrebs, Weichteilsarkom, Retinoblastom, Dünndarmkrebs, Magenkrebs, Thymuskrebs, Schilddrüsenkrebs, Trophoblastkrebs, Blasenmole, Gebärmutterkrebs, Endometriumkrebs, Vaginakrebs, Vulvakrebs, Akustikusneurinom, Mycosis fungoides, Insulinom, Karzinoid-Syndrom, Somatostatinom, Zahnfleischkrebs, Herzkrebs, Lippenkrebs, Hirnhautkrebs, Mundkrebs, Nervenkrebs, Gaumenkrebs, Ohrspeicheldrüsenkrebs, Bauchfellkrebs, Rachenkrebs, Brustfellkrebs, Speicheldrüsenkrebs, Zungenkrebs und Tonsillenkrebs.

## Revendications

1. Méthode permettant l'identification d'antigènes spécifiques de tumeurs comprenant :

   i) la dissolution ou la mise en suspension d'un échantillon de tumeur dans un fluide ;
   ii) le passage dudit fluide à travers un dispositif microfluidique pour l'identification d'antigènes spécifiques de tumeurs comprenant : au moins un canal d'écoulement contenant une pluralité de micropiliers agencés en un réseau auquel est fixé au moins une molécule, ou au moins un complexe, qui a lié à celui-ci au moins un anticorps anti-complexe majeur d'histocompatibilité ou au moins un anticorps anti-antigène leucocytaire pan-humain grâce auquel un peptide: complexe majeur d'histocompatibilité (pCMH) dans un échantillon s'écoulant à travers ledit canal pouvant être extrait dudit échantillon à l'aide dudit au moins un anticorps ;
   iii) la liaison audit au moins un anticorps d'au moins un pCMH dans ledit échantillon ;
   iv) le retrait dudit au moins un pCMH lié de la partie iii) dudit dispositif ;
   v) la comparaison dudit peptide dudit pCMH lié avec une bibliothèque d'antigènes dérivés d'agents pathogènes pour déterminer si ledit peptide présente une homologie, au moyen d'une comparaison des structures de séquence, avec au moins un antigène pathogène, ou une partie de celui-ci, et lorsqu'il existe une homologie supérieure à 60 % ;
   vi) l'identification dudit peptide en tant qu'antigène spécifique de tumeur destiné à être utilisé dans une cancérothérapie.

2. Méthode selon la revendication 1, ladite bibliothèque d'antigènes dérivés d'agents pathogènes comprenant une bibliothèque organisée d'antigènes pathogènes connus.

3. Méthode selon les revendications 1 ou 2, lesdits antigènes pathogènes étant des antigènes pathogènes humains.

4. Méthode selon l'une quelconque des revendications 1 à 3, lesdits antigènes pathogènes étant des virus.

5. Méthode selon l'une quelconque des revendications 1 à 4, lesdits antigènes pathogènes étant dérivés d'au moins un virus, y compris toute combinaison de ceux-ci, choisi dans le groupe comprenant :
   Abyssoviridae ; Ackermannviridae ; Actantavirinae ; Adenoviridae ; Agantavirinae ; Aglimvirinae ; Alloherpesviridae ; Alphaflexiviridae ; Alphaherpesvirinae ; Alphairidovirinae ; Alphasatellitidae ; Alphatetraviridae ; Alvernaviridae ; Amalgaviridae; Amnoonviridae ; Ampullaviridae ; Anelloviridae ; Arenaviridae ; Arquatrovirinae ; Arteriviridae ; Artoviridae ; Ascoviridae ; Asfarviridae ; Aspiviridae ; Astroviridae ; Autographivirinae ; Avsunviroidae ; Avulavirinae ; Bacilladnaviridae ; Baculoviridae ; Barnaviridae; Bastillevirinae ; Bclasvirinae ; Belpaoviridae ; Benyviridae ; Betaflexiviridae; Betaherpesvirinae ; Betairidovirinae ; Bicaudaviridae ; Bidnaviridae ; Birnaviridae ; Bornaviridae ; Botourmiaviridae ; Brockvirinae ; Bromoviridae ; Bullavirinae ; Caliciviridae ; Calvusvirinae ; Carmotetraviridae ; Caulimoviridae ; Ceronivirinae ; Chebruvirinae ; Chordopoxvirinae ; Chrysoviridae ; Chuviridae ; Circoviridae ; Clavaviridae ; Closteroviridae ; Comovirinae ; Coronaviridae ; Corticoviridae ; Crocarterivirinae ; Cruliviridae ; Crustonivirinae ; Cvivirinae ; Cystoviridae ; Dclasvirinae ; Deltaflexiviridae ; Densovirinae ; Dicistroviridae ; Endornaviridae ; Entomopoxvirinae ; Equarterivirinae ; Eucampyvirinae ; Euroniviridae ; Filoviridae ; Fimoviridae ;

Firstpapillomavirinae ; Flaviviridae ; Fuselloviridae ; Gammaflexiviridae ; Gammaherpesvirinae ; Geminialphasatellitinae ; Geminiviridae ; Genomoviridae ; Globuloviridae ; Gokushovirinae ; Guernseyvirinae ; Guttaviridae ; Hantaviridae ; Hepadnaviridae ; Hepeviridae ; Herelleviridae ; Heroarterivirinae ; Herpesviridae ; Hexponivirinae ; Hypoviridae ; Hytrosaviridae ; Iflaviridae ; Inoviridae ; Iridoviridae ; Jasinkavirinae ; Kitaviridae ; Lavidaviridae ; Leishbuviridae ; Letovirinae ; Leviviridae ; Lipothrixviridae ; Lispiviridae ; Luteoviridae ; Malacoherpesviridae ; Mammantavirinae ; Marnaviridae ; Marseilleviridae ; Matonaviridae ; Mccleskeyvirinae ; Mclasvirinae ; Medioniviridae ; Medionivirinae ; Megabirnaviridae; Mesoniviridae ; Metaparamyxovirinae ; Metaviridae ; Microviridae ; Mimiviridae ; Mononiviridae ; Mononivirinae ; Mymonaviridae ; Myoviridae ; Mypoviridae ; Nairoviridae ; Nanoalphasatellitinae ; Nanoviridae ; Narnaviridae ; Nclasvirinae ; Nimaviridae ; Nodaviridae ; Nudiviridae ; Nyamiviridae ; Nymbaxtervirinae ; Okanivirinae ; Orthocoronavirinae ; Orthomyxoviridae ; Orthoparamyxovirinae ; Orthoretrovirinae ; Ounavirinae ; Ovaliviridae ; Papillomaviridae ; Paramyxoviridae ; Partitiviridae ; Parvoviridae ; Parvovirinae ; Pclasvirinae ; Peduovirinae ; Peribunyaviridae ; Permutotetraviridae ; Phasmaviridae ; Phenuiviridae ; Phycodnaviridae ; Picobirnaviridae ; Picornaviridae ; Picovirinae ; Piscanivirinae ; Plasmaviridae ; Pleolipoviridae ; Pneumoviridae ; Podoviridae ; Polycipiviridae ; Polydnaviridae ; Polyomaviridae ; Portogloboviridae ; Pospiviroidae ; Potyviridae ; Poxviridae ; Procedovirinae ; Pseudoviridae ; Qinviridae ; Quadriviridae; Quinvirinae ; Regressovirinae ; Remotovirinae ; Reoviridae ; Repantavirinae ; Retroviridae ; Rhabdoviridae ; Roniviridae; Rubulavirinae ; Rudiviridae ; Sarthroviridae ; Secondpapillomavirinae ; Secoviridae ; Sedoreovirinae ; Sepvirinae ; Serpentovirinae ; Simarterivirinae ; Siphoviridae ; Smacoviridae ; Solemoviridae ; Solinviviridae ; Sphaerolipoviridae ; Spinareovirinae ; Spiraviridae ; Spounavirinae ; Spumaretrovirinae ; Sunviridae ; Tectiviridae ; Tevenvirinae ; Tiamatvirinae ; Tobaniviridae ; Togaviridae ; Tolecusatellitidae ; Tombusviridae ; Torovirinae ; Tospoviridae ; Totiviridae ; Tristromaviridae ; Trivirinae ; Tunavirinae ; Tunicanivirinae ; Turriviridae ; Twortvirinae ; Tymoviridae ; Variarterivirinae ; Vequintavirinae ; Virgaviridae ; Wupedeviridae ; Xinmoviridae ; Yueviridae ; et Zealarterivirinae.

6.  Méthode selon l'une quelconque des revendications 1 à 5, ledit antigène pathogène étant un antigène de cytomégalovirus ou un antigène du virus d'Epstein-Barr ou un antigène de virus de l'herpès, ou un antigène de poxvirus, ou un antigène d'hépadnavirus, ou un antigène de virus de la grippe, ou un antigène de coronavirus, ou un antigène de virus d'hépatite, ou un antigène de VIH, ou un antigène de bunyavirus.

7.  Méthode selon l'une quelconque des revendications 1 à 6, ladite comparaison dudit peptide dudit pCMH lié avec une bibliothèque d'antigènes pathogènes impliquant une notation peptidique et/ou une notation d'alignement pour déterminer ladite homologie.

8.  Méthode selon l'une quelconque des revendications 1 à 7, ladite comparaison dudit peptide dudit pCMH lié avec une bibliothèque d'antigènes pathogènes impliquant la détermination :

    a) de la similitude ou de l'identité de l'ensemble des structures de séquence dudit peptide et desdits antigènes pathogènes ; et/ou
    b) de la similitude ou de l'identité des acides aminés clés dans les sites de liaison clés dudit peptide et desdits antigènes pathogènes ; et/ou
    c) du plus grand nombre d'acides aminés clés similaires ou identiques dans les sites de liaison clés dudit peptide et desdits antigènes pathogènes.

9.  Dispositif d'identification d'antigènes spécifiques de tumeurs comprenant :

    un dispositif microfluidique comprenant au moins un canal d'écoulement contenant une pluralité de micropiliers agencés en un réseau auquel est fixé au moins une molécule, ou au moins un complexe, qui a lié à celui-ci au moins un anticorps anti-complexe majeur d'histocompatibilité ou au moins un anticorps anti-antigène leucocytaire pan-humain (HLA) grâce auquel un peptide:complexe majeur d'histocompatibilité (pCMH) dans un échantillon s'écoulant à travers ledit canal peut être extrait dudit échantillon à l'aide dudit au moins un anticorps ; et
    un processeur adapté à l'identification dudit peptide lié audit complexe majeur d'histocompatibilité et à la comparaison dudit peptide dudit pCMH lié avec une bibliothèque d'antigènes peptidiques dérivés d'agents pathogènes de manière à déterminer si ledit peptide lié ou qui était lié audit complexe majeur d'histocompatibilité présente une homologie, au moyen d'une comparaison des structures de séquence, avec au moins un antigène pathogène, ou une partie de celui-ci, et lorsqu'il existe une homologie supérieure à 60 % ; indiquant ledit peptide lié ou qui était lié audit complexe majeur d'histocompatibilité en tant qu'antigène spécifique de tumeur pour une utilisation en cancérothérapie.

**10.** Dispositif selon la revendication 9, ledit anticorps anti-pan-HLA étant choisi dans le groupe comprenant : les CMH de classe I A, B et C.

**11.** Dispositif selon la revendication 9, ledit anticorps anti-pan-HLA étant choisi dans le groupe comprenant : les CMH de classe II DP, DM, DO, DQ et DR.

**12.** Dispositif selon l'une quelconque des revendications 9 à 11, ledit anticorps étant anti-humain.

**13.** Dispositif selon l'une quelconque des revendications 9 à 12, ladite molécule ou ledit complexe étant un complexe de groupes fonctionnels thiol et alcène.

**14.** Dispositif selon l'une quelconque des revendications 9 à 13, ladite molécule ou ledit complexe comprenant de la biotine et de la streptavidine.

**15.** Dispositif selon l'une quelconque des revendications 9 à 14, ladite molécule ou ledit complexe comprenant plus d'un anticorps lié à la streptavidine.

**16.** Méthode de stratification de patients pour un traitement de cancer par inhibiteur de point de contrôle comprenant :

i) la dissolution ou la mise en suspension d'un échantillon de tumeur d'un patient dans un fluide ;
ii) le passage dudit fluide à travers un dispositif microfluidique pour l'identification d'antigènes spécifiques de tumeurs comprenant : au moins un canal d'écoulement contenant une pluralité de micropiliers agencés en un réseau auquel est fixé au moins une molécule, ou au moins un complexe, qui a lié à celui-ci au moins un anticorps anti-complexe majeur d'histocompatibilité ou au moins un anticorps anti-antigène leucocytaire pan-humain grâce auquel un peptide: complexe majeur d'histocompatibilité (pCMH) dans un échantillon s'écoulant à travers ledit canal pouvant être extrait dudit échantillon à l'aide dudit au moins un anticorps ;
iii) la liaison audit au moins un anticorps d'au moins un pCMH dans ledit échantillon ;
iv) le retrait dudit au moins un pCMH lié de la partie iii) dudit dispositif ;
v) la comparaison dudit peptide dudit pCMH lié avec une bibliothèque d'antigènes pathogènes humains pour déterminer si ledit peptide présente une homologie, au moyen d'une comparaison des structures de séquence, avec au moins un antigène pathogène humain, ou une partie de celui-ci, et lorsqu'il existe une homologie supérieure à 60 % ;
vi) l'identification dudit peptide en tant qu'antigène spécifique de tumeur ; et
vii) lorsque ledit peptide est trouvé, la stratification dudit patient comme approprié au traitement en utilisant une quantité efficace d'au moins un inhibiteur de point de contrôle.

**17.** Méthode selon la revendication 16, ledit cancer étant choisi dans le groupe comprenant : le cancer du nasopharynx, le cancer de la synovie, le cancer hépatocellulaire, le cancer du rein, le cancer des tissus conjonctifs, un mélanome, le cancer du poumon, le cancer de l'intestin, le cancer du côlon, le cancer du rectum, le cancer colorectal, le cancer du cerveau, le cancer de la gorge, le cancer de la bouche, le cancer du foie, le cancer des os, le cancer du pancréas, un choriocarcinome, un gastrinome, un phéochromocytome, un prolactinome, une leucémie/un lymphome à lymphocytes T, un névrome, la maladie de von Hippel-Lindau, le syndrome de Zollinger-Ellison, le cancer de la glande surrénale, le cancer de l'anus, le cancer des voies biliaires, le cancer de la vessie, le cancer de l'urètre, un oligodendrogliome, un neuroblastome, un méningiome, une tumeur de la moelle épinière, un ostéochondrome, un chondrosarcome, un sarcome d'Ewing, le cancer d'un site primaire inconnu, un carcinoïde, un carcinoïde du tractus gastro-intestinal, un fibrosarcome, le cancer du sein, la maladie de Paget, le cancer du col de l'utérus, le cancer de l'œsophage, le cancer de la vésicule biliaire, le cancer de la tête, le cancer de l'œil, le cancer du cou, le cancer du rein, la tumeur de Wilms, le cancer du foie, le sarcome de Kaposi, le cancer de la prostate, le cancer des testicules, la maladie de Hodgkin, un lymphome non hodgkinien, le cancer de la peau, un mésothéliome, un myélome multiple, le cancer de l'ovaire, le cancer du pancréas endocrinien, un glucagonome, le cancer de la parathyroïde, le cancer du pénis, le cancer de l'hypophyse, un sarcome des tissus mous, un rétinoblastome, le cancer de l'intestin grêle, le cancer de l'estomac, le cancer du thymus, le cancer de la thyroïde, le cancer trophoblastique, une môle hydatiforme, le cancer de l'utérus, le cancer de l'endomètre, le cancer du vagin, le cancer de la vulve, un névrome acoustique, une mycose fongoïde, un insulinome, le syndrome carcinoïde, un somatostatinome, le cancer des gencives, le cancer du cœur, le cancer de la lèvre, le cancer des méninges, le cancer de la bouche, le cancer des nerfs, le cancer du palais, le cancer de la glande parotide, le cancer du péritoine, le cancer du pharynx, le cancer de la plèvre, le cancer des glandes salivaires, le cancer de la langue et le cancer des amygdales.

Figure 1A

Figure 1B

Figure 2B

Figure 2D

Figure 2A

Figure 2C

Figure 3A

Figure 3B

Figure 3C

Figure 3D

Figure 3E

Figure 4A

Motif Reference

Group 1 of 2

n=553

Group 2 of 2

n=236

Kullback Lieber distance

Number of clusters

Figure 4B

36

Figure 4B (Continued)

Figure 5A

Figure 5B

INPUT:
List of 8-12aa long peptides

HEX

Use BLAST to find similar sequences

Custom Viral Proteomes Database

Custompositional weighted alignment to favor similarity in the TCR interaction area

Filter the candidates for binding affinity to up to 6 HLA

OUTPUT:
tumor peptides with high HLA-binding affinity and high viral homology

Figure 6

A

Viral Peptides similar to TYR

Viral Peptides similar to gp100

Viral Peptides similar to TRP2

-14    -7    0    Tumor growth
                                    Days

First immunization    Boost    Tumor injection    Endpoint

B

**Tumor Volumes**

Mock
TYR1 pool
TRP2 pool
gp100 pool

** **** ****    *** **

C

**Viral Pool 1 - similar to TYR1**

**Viral Pool 2 - similar to TRP2**

**Viral Pool 3 - similar to gp100**

Figure 7

41

Figure 8A

Figure 8B

C

D

E  B16 F10

Mock    Uncoated Virus    Viral PeptiCRAd    TRP2 PeptiCRAd

F

G

Figure 8

Patient CMV+

Figure 9A

PBMCs pulsed with NLVPMVATV

Figure 9B

| | S1 | S2 | S3 | S4 | S5 |
|---|---|---|---|---|---|
| Weight | 0.06gr | 0.05g | 0.05g | 0.01g | 0.02g |
| Total number of peptides | 935 | 715 | 172 | 1155 | 260 |
| Unique peptides (no duplicates peptides) | 916 | 695 | 172 | 1128 | 256 |
| 7-13mers | 689 (75%) | 580 (83%) | 124 (72%) | 924 (82%) | 201 (79%) |

Figure 10A

Figure 10B

Figure 11A

## ccRCC

## Bladder tumour

Figure 11B

Figure 12A

| Peptide | Uniprot ID | Gene name | |
|---------|-----------|-----------|---|
| KKKKKKSYLPPGTSL | Q8VCF0 | MAVS | PeptiCRAd1 |
| KKKKKKRYLPAPTAL | Q9JL70 | FANCA | |
| KKKKKKYIPAARHL | O54692 | ZW10 | PeptiCRAd2 |
| KKKKKKLYKESLSRL | Q6URW6-2 | MYH14 | |
| KKKKKKYLNVREAV | Q8R3J5 | Chac1 | PeptiCRAd3 |
| KKKKKKFYATIIHDL | Q9EQH7 | Ndst3 | |

Figure 12B

Figure 13

# Tumor-specific peptides

List of tumor peptides

HƎX

Reference Peptide

Matrix

QNTSGACPMVILYFWHKRED

Curated viral protein database

List of viral peptides

- B-Score
- Positional Weighted Alignment Score
- MHCI Binding prediction

Figure 13 Continued

## REFERENCES CITED IN THE DESCRIPTION

### Non-patent literature cited in the description

- **CAPASSO et al.** *Annals of Oncology*, 01 December 2017, vol. 28, 11
- **KIM et al.** *BMC Bioinformatics*, 30 November 2009, vol. 10, 394
- **KIM Y ; SIDNEY J ; PINILLA C ; SETTE A ; PETERS B**. *Derivation of an amino acid similarity matrix for peptide: MHC binding and its application as a Bayesian prior.*
- **KIM et al.** *BMC Bioinformatics*, 30 November 2009
- **KIM Y ; SIDNEY J ; PINILLA C ; SETTE A ; PETERS B.** *Derivation of an amino acid similarity matrix for peptide: MHC binding and its application as a Bayesian prior*
- **KIM Y ; SIDNEY J ; PINILLA C ; SETTE A ; PETERS B.** Derivation of an amino acid similarity matrix for peptide: MHC binding and its application as a Bayesian prior.. *BMC Bioinformatics*, 30 November 2009, vol. 10, 394
- **C. CAPASSO ; M. HIRVINEN ; M. GAROFALO ; D. ROMANIUK ; L. KURYK ; T. SARVELA ; A. VITALE ; M. ANTOPOLSKY ; A. MAGARKAR ; T. VIITALA**. Oncolytic adenoviruses coated with MHC-I tumor epitopes increase the antitumor immunity and efficacy against melanoma. *Oncoimmunology*, 2016, vol. 5, e1105429
- **D. WEINSTEIN ; J. LEININGER ; C. HAMBY ; B. SAFAI**. Weinstein 2014 Diagnostic and Prognostic biomarkers in melanoma. *J. Clin. Aesthet. Dermatol.*, 2014, vol. 7, 13-24
- **J. SIDNEY ; E. ASSARSSON ; C. MOORE ; S. NGO ; C. PINILLA ; A. SETTE ; B. PETERS**. Quantitative peptide binding motifs for 19 human and mouse MHC class i molecules derived using positional scanning combinatorial peptide libraries. *Immunome Res*, 2008, vol. 4, 2
- **A. K. SHARMA ; J. J. KUHNS ; S. YAN ; R. H. FRIEDLINE ; B. LONG ; R. TISCH ; E. J. COLLINS**. Class I Major Histocompatibility Complex Anchor Substitutions Alter the Conformation of T Cell Receptor Contacts. *J. Biol. Chem.*, 2001, vol. 276, 21443-21449
- **M. ANDREATTA ; M. NIELSEN**. Gapped sequence alignment using artificial neural networks: Application to the MHC class i system. *Bioinformatics*, 2016, vol. 32, 511-517
- **P. C. ROSATO ; S. WIJEYESINGHE ; J. M. STOLLEY ; C. E. NELSON ; R. L. DAVIS ; L. S. MANLOVE ; C. A. PENNELL ; B. R. BLAZAR ; C. C. CHEN ; M. A. GELLER**. Virus-specific memory T cells populate tumors and can be repurposed for tumor immunotherapy. *Nat. Commun.*, 2019, vol. 10, 567
- **S. GIGUÈRE ; A. DROUIN ; A. LACOSTE ; M. MARCHAND ; J. CORBEIL ; F. LAVIOLETTE**. MHC-NP: Predicting peptides naturally processed by the MHC. *J. Immunol. Methods*, 2013, vol. 400-401, 30-36
- **BASSANI-STERNBERG M ; PLETSCHER-FRANKILD S ; JENSEN LJ ; MANN M**. Mass spectrometry of human leukocyte antigen class I peptidomes reveals strong effects of protein abundance and turnover on antigen presentation.. *Mol Cell Proteomics.*, 2015, vol. 14 (3), 658-73
- **LEE SH ; HU W ; MATULAY JT ; SILVA MV ; OWCZAREK TB ; KIM K et al.** Tumor Evolution and Drug Response in Patient-Derived Organoid Models of Bladder Cancer. *Cell*, 2018, vol. 173 (2), 515-28
- **PURCELL AW ; RAMARATHINAM SH ; TERNETTE N.** Mass spectrometry-based identification of MHC-bound peptides for immunopeptidomics.. *Nat Protoc.*, 2019, vol. 14 (6), 1687-707
- **JURTZ V ; PAUL S ; ANDREATTA M ; MARCATILI P ; PETERS B ; NIELSEN M**. NetMHCpan-4.0: Improved Peptide-MHC Class I Interaction Predictions Integrating Eluted Ligand and Peptide Binding Affinity Data. *J Immunol.*, 2017, vol. 199 (9), 3360-8
- **NIELSEN M ; ANDREATTA M.** NetMHCpan-3.0; improved prediction of binding to MHC class I molecules integrating information from multiple receptor and peptide length datasets.. *Genome Med*, 2016, vol. 8 (1), 33
- **HOOF I ; PETERS B ; SIDNEY J ; PEDERSEN LE ; SETTE A ; LUND O et al.** NetMHCpan, a method for MHC class I binding prediction beyond humans.. *Immunogenetics.*, 2009, vol. 61 (1), 1-13